(19) Europäisches Patentamt
European Patent Office
Office européen des brevets

(11) Publication number: **0 504 423 A1**

(12) # EUROPEAN PATENT APPLICATION published in accordance with Art. 158(3) EPC

(21) Application number: **91917334.4**

(22) Date of filing: **03.10.91**

(86) International application number: **PCT/JP91/01330**

(87) International publication number: **WO 92/06377 (16.04.92 92/09)**

(51) Int. Cl.[5]: **G01N 33/53**, G01N 33/577, G01N 33/543, C12P 21/08, C12N 15/06, C12N 5/20

(30) Priority: **04.10.90 JP 264935/90**
**20.06.91 JP 174768/91**

(43) Date of publication of application: **23.09.92 Bulletin 92/39**

(84) Designated Contracting States: **AT BE CH DE ES FR GB IT LI NL SE**

(71) Applicant: **TEIJIN LIMITED**
**6-7, Minamihonmachi 1-chome Chuo-ku**
**Osaka-shi Osaka 541(JP)**

(72) Inventor: **HOSODA, Kenji**
**4-7-26-306, Sennin-cho**
**Hachioji-shi, Tokyo 193(JP)**
Inventor: **HONDA, Hitomi**
**Teijin Ozu Apatp, 28, Ozu-cho 1-chome**
**Iwakuni-shi, Yamaguchi 740(JP)**
Inventor: **KUBOTA, Takaharu**
**3-18-4-211, Tamadaira**
**Hino-shi, Tokyo 191(JP)**
Inventor: **NAKAMOTO, Tadakatsu**
**3-18-4-115, Tamadaira**
**Hino-shi, Tokyo 191(JP)**

(74) Representative: **Kraus, Walter, Dr.**
**Patentanwälte Kraus, Weisert & Partner**
**Thomas-Wimmer-Ring 15**
**W-8000 München 22(DE)**

(54) **METHOD AND KIT FOR IMMUNOASSAY OF PROPEPTIDE OF OSTEOCALCIN AND PROOSTEOCALCIN.**

(57) (i) A method for the immunoassay of the propeptide of osteocalcin present in a specimen by the sandwich method, wherein an antibody which specifically recognizes said propeptide is used as an antibody (first antibody) combined with an insoluble carrier and a labelled antibody (second antibody), and a kit therefor; (ii) a method for the immunoassay of proosteocalcin present in a specimen by the sandwich method, wherein one of an antibody (first antibody) combined with an insoluble carrier and a labelled antibody (second antibody) is an antibody which specifically recognizes the propeptide of osteocalcin, while the other is an antibody which specifically recognizes osteocalcin, and a kit therefor; and (iii) a method for the immunoassay of the total content of the propeptide of osteocalcin and proosteocalcin in a specimen by the competitive method, wherein an antibody which specifically recognizes the propeptide of osteocalcin is used as an antibody which combines with an insoluble carrier or which can be insolubilized.

EP 0 504 423 A1

Industrially Applicable Field

This invention relates to a method for immunologically measuring the propeptide of osteocalcin, proosteocalcin or both of them in a sample to be inspected, and a kit therefore. More specifically this invention relates to a method and a kit for measuring selectively and in high sensitivity the propeptide of osteocalcin or proosteocalcin in an inspection sample, and further to a method and a kit for measuring the total quantity of the propeptide of osteocalcin and proosteocalcin in an inspection sample.

In the present specification, the following terms are defined as meanings explained about each of them.

(a) Propeptide of osteocalcin:

A propeptide sequence being located at the amino terminus side (the 5'-side) of the amino acid sequence of osteocalcin in an osteoblast (being composed of a sequence of 26 amino acids in case of human osteocalcin)

(b) Proosteocalcin:

An amino acid sequence composed of the above propeptide and osteocalcin (in case of human osteocalcin being composed of a sequence of 26 amino acids based on the propeptide and a sequence of 49 amino acids based on the osteocalcin)

(c) Osteocalcin:

The amino acid sequence of osteocalcin (being composed of a sequence of 49 amino acids in case of human osteocalcin)

(d) Preproosteocalcin:

An amino acid sequence composed of prepeptide, propeptide and osteocalcin (an amino acid sequence comprising the above proosteocalcin to which, at the amino terminus side (5'-side), the prepeptide is linked; in case of human preproosteocalcin, being composed of a sequence of 23 amino acids based on the prepeptide, a sequence of 26 amino acids based on the propeptide and a sequence of 49 amino acids based on the osteocalcin)

Prior Art

Osteocalcin (an alias, bone gla protein (BGP)) is a protein capable of binding to bone calcium depending on vitamin K. Osteocalcin has a molecular weight of 5,800 and is constituted by 49 amino acid residues. This protein is produced by osteoblasts and amounts to about 20% of the constitutive components of bone noncollagen proteins. This protein has $\gamma$-carboxyglutamic acid residues and strong affinity for hydroxyapatite, and is therefore surmised to play an important role in formation of bone matrix.

This osteocalcin was first found in the bones of chickenes and cattle (Proc. Natl. Acad. Sci. USA, 72, 3925 (1975), ibid. 73, 1447 (1976)), and thereafter found in the bones of various animals including human beings (J.B.C. 255, 8685 (1980)), and their analogy in structure is pointed out.

Osteocalcin has, in osteoblasts, a structure of m-RNA constituted by three parts of ① the prepeptide sequence having a secretory function, ② the propeptide sequence being the site linking to $\gamma$-carboxylase, and ③ the mature osteocalcin sequence, a Gla protein in this order. The following is surmized. Namely, m-RNA is read, in ribosome, into preproosteocalcin protein, an osteocalcin, precursor. Then, after the cleavage of the prepeptide sequence, the osteocalcin precursor proosteocalcin is linked, though its propeptide part, to $\gamma$-carboxylase in the Golgi bodies, and all or part of the Glu residues in the proosteocalcin amino acid sequence are converted to Gla residues. Then, the propeptide and osteocalcin are detached with the peptidase, and the propeptide is decomposed in the cell and osteocalcin is mainly secreted.

As for the propeptide sequence of osteocalcin, A.J. Celeste et al. reported the cDNA sequence and amino acid sequence of the preproosteocalcin (EMBO J. 5, 1885-1890 (1986)).

Problems to be Solved by the Invention

In the Japan Bone Metabolism Society held on July 20, 1990, Kasai et al. announced a method to

measure the propeptide of osteocalcin by a competition method using an immobilized antigen, and therein the measurement values of the propeptide are higher in adults and shows a positive correlation with age. However, in consideration of osteogenesis ability lowering in general in proportion to age, this announced measurement results are contradictory to a surmise that this marker (propeptide) indicates osteogenesis ability. As a reason of occurrence of this contradiction, there can be mentioned imperfection of the measurement system. One of the reasons of imperfection is thought to lie in lowness of the specificity of the antibody used, and another is thought to lie in lowness of the specificity of the measurement system used.

The reason to use the competition method in the measurement system of the propeptide is surmised to be that it was predicted that such a small peptide (26 amino acids, molecular weight about 3,000) was too small to measure by a sandwich method and thus it was generally difficult to measure it by the sandwich method using a large antibody (molecular weight about 150,000).

Heretofore, an immunological measurement method showing high specificity, quantitativeness and reproducibility has not yet been provided on the propeptide of osteocalcin, which can be an indicator of the state of osteogenesis. In addition to the propeptide of osteocalcin, a method to immunologically measure proosteocalcin in high sensitivity is not provided, either.

The quantity of the propeptide of osteocalcin or proosteocalcin in an inspection sample is generally extremely small (for example, about 20 to about 30 ng in case of the propeptide), and it requires an extremely highly sensitive measurement system to measure the quantity of them specifically and quantitatively.

Thus, the first object of this invention lies in providing a method and a kit for specifically measuring the propeptide of osteocalcin in an inspection sample by an immunological measurement means based on a sandwich method.

The second object of the invention lies in providing a method and a kit for specifically measuring proosteocalcin in an inspection sample by an immunological measurement means based on a sandwich method.

The third object of the invention lies in providing a method and a kit for specifically measuring the total quantity of the propeptide of osteocalcin and proosteocalcin in an inspection sample of an immunological measurement means based on a competition method.

Another object of the invention lies in providing a method for diagnosing the state of osteogenesis based on the results of the above immunological measurement.

Still another object of the invention lies in providing, in the above immunological measurement means, a method and a kit for highly sensitive measurement, excellent in quantitativeness and reproducibility.

Still another object of the invention lies in providing polyclonal antibodies and monoclonal antibodies usable in the above various measurement methods.

Still other objects of the invention will be apparent from the following description.

## Means for Solving the problems

According to the studies of the present inventors, it was found that the above objects and advantages of this invention can fundamentally be attained by the following method I, method II and method III. Further, according to this invention are provided respective kids corresponding to these methods.

### Method I:

A method for immunologically measuring the propeptide of osteocalcin in an inspection sample by a sandwich method, wherein antibodies specifically recognizing the propeptide of osteocalcin are used as the antibody linked to the insoluble carrier (the first antibody) and the labeled antibody (the second antibody).

According to this method I, the propeptide of osteocalcin in an inspection sample can specifically be measured, whereas proosteocalcin is not recognized and thus not measured.

According to this method, suprisingly, the propeptide of osteocalcin (having a sequence of 26 amino acids in case of human propeptide), a small peptide, can be measured in high sensitivity using antibodies, especially identical polyclonal antibodies and can be measured without recognition of proosteocalcin. According to this method I, this proosteocalcin was not recognized although the propeptide sequence was contained in the molecule. Although it is uncertain why the propeptide of osteocalcin is specifically measured, the reason probably seems to be that osteocalcin in proosteocalcin inhibits the formation of sandwiches utilizing the second antibody.

Since the propeptide of osteocalcin can be measured specifically and in high sensitivity, it becomes

possible be diagnose the state of osteogenesis by inspecting the concentration of the propeptide.

Method II:

A method for immunologically measuring proosteocalcin in an inspection sample by a sandwich method, wherein any one of the antibody linked to the insoluble carrier (the fist antibody) and the labeled antibody (the second antibody) is an antibody specifically recognizing the propeptide of osteocalcin, and the other antibody is an antibody specifically recognizing osteocalcin.

By this method II, proosteocalcin in an inspection sample can specifically be measured. The propeptide of osteocalcin cannot be measured by this method II.

Method III:

A method for immunologically measuring the total quantity of the propeptide of osteocalcin and proosteocalcin in an inspection sample by a competition method, wherein an antibody specifically recognizing the propeptide of osteocalcin is used as the antibody liking to the insoluble carrier.

According to this method III, the total quantity of the propeptide of osteocalcin and proosteocalcin in an inspection sample is measured. Namely, the quantity of all the propeptide of osteocalcin in the inspection sample is measured.

Further, according to this invention, the following antibodies used in the above method I, method II and method III are provided.

(i) a polyclonal antibody specifically recognizing the propeptide of human osteocalcin

(ii) a polyclonal antibody specifically recognizing the sequence of the 14-position to the 26-position in the amino acid sequence of the propeptide of human osteocalcin.

(iii) a polyclonal antibody specifically recognizing the sequence of the 1-position to the 13-position in the amino acid sequence of the propeptide of human osteocalcin

(iv) a polyclonal antibody specifically recognizing the 14-position to the 26-position in the amino acid sequence of the propeptide of human osteocalcin

As this monoclonal antibody, there can be used one produced by the later-described hybridoma 3F9 or 4E12.

Methods for preparing antibodies specifically recognizing the propeptide of osteocalcin used in the above method I, method II and method III are described below. Although description is, specifically, made on antibodies specifically recognizing the propeptide of human osteocalcin, antibodies can also be prepared in the same manner on the propeptides, other than the propeptide of human osteocalcin, namely of the osteocalcin of rats, mice or dogs other than human being.

1) Synthesis of the propeptide of human osteocalcin (Preparation of an antigen)

The propeptide of human osteocalcin, represented by the following formula [I], was synthesized using a peptide synthesizer produced by ABI Co.

$H_2N$-Lys-Pro-Ser-Gly-Ala-Glu-Ser-Ser-Lys-Ala-
Phe-Val-Ser-Lys-Gln-Glu-Gly-Ser-Glu-Val-Val-
Lys-Arg-Pro-Arg-Arg-COOH .... [I]

Hereinafter, the name of this peptide is abbreviated as "Ostpro-26".

2) Preparation of an immunogen

The following three methods can be utilized for preparation of an immunogen of Ostpro-26. ① physicochemical mixing with a synthetic high polymer such as PVP, ② chemical bond to a carrier protein (KLH, BSA or the like), ③ bond to (adsorption on or chemical bond to) particles such as liposome or cells.

3) Immunization method (preparation of antibodies)

An animal (sheep, goat, rabbit, rat, mouse, chicken or the like) is immunized with the above-prepared

immunogen. As a usable adjuvant, there can be mentioned a complete Freund's adjuvant, an incomplete Freund's adjuvant, $Al(OH)_3$ or the like. After increase of antibody titer, serum containing antibodies is recovered and the antibodies are purified. Thus is obtained a polyclonal antibody specifically recognizing the propeptide of human osteocalcin (hereinafter, this antibody is sometimes abbreviated as "ap-26").

Further, in the same manner as in the above 1) to 3) were synthesized in N-terminal 13 residues ($H_2N$-Lys-Pro-Ser-Gly-Ala-Glu-Ser-Ser-Lys-Ala-Phe-Val-Ser-) and C-terminal 13 residues (Lys-Glu-Glu-Gly-Ser-Glu-Val-Val-Lys-Arg-Pro-Arg-Arg-COOH), respectively, and polyclonal antibodies were obtained corresponding to them, respectively. The polyclonal antibody against the above peptide of the N-terminal 13 residues is abbreviated as "apN-13", and the polyclonal antibody against the peptide of the C-terminal 13 residued is abbreviated as "apC-13".

Further, the monoclonal antibody against the peptide of the C-terminal 13 residues of the above propeptide of human osteocalcin (the amino acid sequence of from the 14-position to the 26-position) was prepared according to the method of Keller and Milstein. Namely, a Balb/C mouse was immunized with a KLH conjugated of the peptide of the C-terminal 13 residues, and hybridomas 3F9 and 4E12 were selected as antibody-producing clones. Antibodies produced by them were equally IgG1. These monoclonal antibodies are abbreviated "M-3F9" and "M-4E12", respectively.

Hereafter, more specific description is made on the immunological measurement method and the kit therefor in this invention.

Inspection samples to be used in the measurement in the above: method I, method II and method III can be any samples either containing the propeptide of osteocalcin, proosteocalcin or both of them, or being expected to contain it. Examples of such samples are body fluids of human beings or animals such as, for example, sera, plasmas and urines; culture broths or culture layers themselves of tissues deriving from bones of human beings or animals such as osteoblasts and bone cells. Sera are most preferably used. Further, such an inspection sample can also be either a culture broth containing the propeptide and/or proosteocalcin each obtained by genetic recombination technique, or a product obtained by treating the culture broth.

In the above method I, as the antibody linked to the insoluble carrier (the first antibody) and the labeled antibody (the second antibody) are used antibodies specifically recognizing the propeptide of osteocalcin. In this occasion, it is preferred that (i) both of the first antibody and the second antibody are polyclonal antibodies (ap-26) specifically recognizing the propeptide of osteocalcin, or that (ii) any one of the first antibody and the second antibody is a polyclonal antibody (ap-26) specifically recognizing the propeptide of osteocalcin, and the other is a polyclonal antibody (apc-13) or monoclonal antibody specifically recognizing the 14-position to 26-position in the amino acid sequence of the propeptide of osteocalcin.

On the other hand, in the above method II, as the antibody specifically recognizing the propeptide of osteocalcin used as any one of the first antibody and the second antibody, polyclonal antibody (ap-26, apN-13 or apC-13) is preferably used and ap-26 or apC-13 is particularly preferred. As the antibody used in this method II and specifically recognizing osteocalcin is preferably used either a polyclonal antibody, or a polyclonal antibody or monoclonal antibody specifically recognizing either the region of the sequence of 20 amino acids at the N-terminus of osteocalcin or the region of the sequence of 14 amino acids at the C-terminus of osteocalcin. The latter two polyclonal antibodies can be prepared by the methods disclosed in Examples 1 and 2 of the PCT application (PCT/JP90/00155).

The above method I and method II are immunological measurement methods usually called sandwich methods, and can be methods known per se except that the above-mentioned antibodies are selected as the first antibody and the second antibody. For example, there can be mentioned the methods described in "KOSO Meneki SokuteiHou (Enzyme Immunoassay), 2nd Edition, written by Eiji Isikawa et al., published by Igaku-Shoin (1982)".

Hereafter, description is made about the case where the antigen contained in an inspection sample is the propeptide of human osteocalcin (method I), but the detail of method II is omitted because procedure itself is the same between the two methods.

In an immunological measurement method using a sandwich method, one of the antibodies against the propeptide of human osteocalcin (the first antibody) is immobilized on a suitable insoluble carrier (e.g., a plastic vessel) (hereinafter, the resultant product is referred to as a "solid phase antibody"). Then, as occasion demands, the surface of the insoluble carrier is covered with a suitable material (e.g., bovine serum albumin) for the purpose of avoiding nonspecific linkage between the insoluble carrier and the reagent or inspection sample to be measured.

The thus obtained insoluble carrier having immobilized the first antibody thereon is contacted and reacted with an inspection sample at a certain temperature for a certain time. In the meantime are linked the solid phase antibody (the first antibody) and an antigen in the inspection sample containing the propeptide

of human osteocalcin. After washing with a suitable washing solution, the propeptide of human osteocalcin linked to the solid phase antibody in the insoluble carrier is contacted with a solution (e.g., an aqueous solution) of the other antibody (the second antibody) of those against the propeptide of human osteocalcin, labeled with a suitable labeling material (e.g., en enzyme) at a certain temperature for a certain time to react the propeptide with the second antibody. After washing the reaction product with a suitable washing solution is measured the quantity of the labeling material labeling the second antibody existing in linkage with the solid phase antibody on the insoluble carrier through the propeptide of human osteocalcin.

The above reactions can also be carried out by simultaneously mixing the solid phase antibody, the labeled antibody and the inspection sample containing the propeptide of human osteocalcin, and simultaneously containing these three members at a certain temperature for a certain time.

The quantity of the propeptide of human osteocalcin in the inspection sample can be calculated from the measured quantity of the labeling substance.

Further, in the competition method of method III, a method can be carried out which comprises labeling the propeptide as an antigen with a solid antibody in an insoluble carrier or an antibody insolubilizable by some method (e.g., use of a second antibody or a crosslinking agent), and making the labeled propeptide compete with the propeptide in an inspection sample.

There was a case, in this invention, where when pure propeptide was added to a human serum specimen, an abnormally high recovery was obtained (400 to 800%). By investigation of this reason, it was revealed that strong interaction between the propeptide and serum proteins brought about this result. Namely, it is considered that the propeptide changes to a constitution easier to recognize. Therefore, it is a preferred embodiment to add animal serum in the range of 2 to 30% to the standard substance, for giving a highly quantitative method for measurement of the propeptide of human osteocalcin.

As for antibodies used in the sandwich methods of this invention (method I and method II), not only IgG but also antibody fragments capable of linking to antigens such as $F(ab')_2$ obtained by digestion of IgG with pepsin, Fab' obtained by reduction of $F(ab')_2$ and Fab obtained by digestion of an antibody with papain can be used as solid antibodies or labeled antibodies obtained by linking labeling materials to them. For example, $F(ab')_2$ is preferred as a labeled antibody.

As insoluble carriers used in the methods for immunological measurement of the propeptide of human osteocalcin, and the like of this invention, there can be exemplified macromolecules such as, for example, polystyrene, polyethylene, polypropylene, polyesters, polyacrylonitrile, fluororesins, crosslinked dextran and polysaccharides; paper; glass; metals; agarose; and their combinations; etc.

Shapes of the insoluble carrier can be various shapes such as, for example, tray shapes, spherical shapes, fibrous shapes, stick shapes, board shapes, vessel shapes, cells and test tubes.

According to the researches of the present inventors, it was found that, in the above methods of this invention for measurement of the propeptide of human osteocalcin in an inspection sample, when as the insoluble carrier is used one having a smooth surface like a mirror surface, nonspecific adsorption of proteins in the inspection sample or the labeled antibody on the carrier is inhibited, and thus measurement sensitivity is hightened and at the same time stability is increased, compared with a carrier having a rough surface.

Heretofore, in order to heighten measurement sensitivity in immunological measurement methods, there have rather generally been used, as the insoluble carriers, those whose surface had been made rough by sanding and whose surface area had thus been made larger. However, in case a substance to be inspected is contained only in an extremely small quantity in an inspection sample, as is the case with the propeptide of human osteocalcin, in proportion as the smoothness of the surface increase, nonspecific adsorption comes to be inhibited and measurement sensitivity increases.

Thus, advantageous as the insoluble carrier is one having a smooth surface like a mirror surface wherein the center line average roughness (Ra) of the surface is 1.5 $\mu$m or less.

Center line average roughness (Ra) means a value of Ra, expressed by a micron unit, given by the following equation when from the roughness curve is extracted a portion of a measurement length of $\ell$ in the direction of its center line, and the center line of the extracted portion, the direction of longitudinal magnification and the roughness curve are expressed by the X axis, the Y axis and $y = f(x)$, respectively:

$$Ra = \frac{1}{\ell}\int_{0}^{R} \left| f(x) \right| dx$$

On this center line average roughness (Ra), description is made in JIS B0601-1982 (Japan), ANSI B46.1-1979 (USA) and R468-1966 (ISO).

In the following examples of this invention, the surface roughnesses of the insoluble carriers were measured using a surface roughness tester Surfcom produced by TOKYO SEIMITSUI CO., LTD. The materials and shapes of the insoluble carriers having a smooth surface is not particularly limited, and those described above are exemplified. A particularly preferred example is polystyrene bead.

Further, it is advantage to use, as the labeling materials of the second antibodies (labeled antibodies), enzymes, fluorescent substances, self-luminous substances, radioactive substances, etc. There can be used peroxidases (HRP), alkaline phosphatases, β-D-galactosidase, etc. as enzymes; fluoresceine isothiocyanate, phycobili proteins, etc. as fluorescent substances; isolucinol, lucigenin, etc. as self-luminous substances; and $^{125}I$, $^{131}I$, $^{14}C$, $^{3}H$, etc. as radioactive substances. However, the labeling materials are not limited to those exemplified, and other ones can be used so long as they can be used in immunological measurement method.

When the labeling material is as enzyme, a substrate and, if necessary, a color former are used for measurement of its activity.

In case peroxides is used as the enzyme, as the substrate is used $H_2O_2$ and as the color former is used 2,2'-azinodi-[3-ethylbenzothiazolinesulfonic acid] ammonium salt (ABTS), 5-aminosalicylic acid, o-phenylenediamine, 4-aminoantipyrin, 3,3'5,5'-tetramethylbenzidine or the like; and in case alkaline phosphatase is used as the enzyme, as the substrate is used o-nitrophenyl phosphate or the like; and in case β-D-galactosidase is used as the enzyme, as the substrate is used fluoresceine-di-(β-D-galactopyranoside), 4-methylumbolliferyl-β-D-galactopyranoside or the like.

A kit used for the above immunological measurement methods of method I and method II is basically composed of a combination of the above-mentioned first antibody (solid phase antibody) and second antibody (labeled antibody).

Thus the kit for immunological measurement of the propeptide of osteocalcin or proosteocalcin comprises a combination of

(a) a solid phase antibody (the first antibody),
(b) a labeled antibody (the second antibody),
(c) a solubilizing agent,
(d) a washing agent
(e) a standard substance and
(f) when a labeled antibody labeled with an enzyme is used, a substrate for measuring enzyme activity and a reaction discontinuation agent,

and the first antibody of the above (a) and the second antibody of the above (b) are selected from those described in method I and method II, respectively.

Further, the solubilizing agents (c) in the kits for immunological measurement of this invention can be those usually used for immunological measurement, and suitable examples thereof are those having a pH in the range of 6.0 to 8.0, including such as, for example, phosphate buffers, tris hydrochloride buffers and acetate buffers. Further1similarly, as washing agents (d), those generally used in immunological measurement are used as such. Examples thereof are physiological saline, phosphate buffers, tris hydrochloride buffers and their mixed solutions. It is possible to add to these washing agents a nonionic surfactant such as Triton x 100, Tween 20 or Brig 35, or an ionic surfactant such as sodium dodecyl sulfate.

According to the method III of this invention, the total quantity of the propeptide of osteocalcin and proosteocalcin in an inspection sample can be measured in high sensitivity. This method III is a method usually called a competition method, and the method itself is known. The antibody used in the method III of this invention is an antibody specifically recognizing the propeptide of osteocalcin, and there can be used those described in the above method I and method II. Specific examples of preferred antibodies are polyclonal antibody (ap-26) and polyclonal antibody (apN-13) specifically recognizing the 1-position to 13-position in the amino acid sequence of the propeptide of osteocalcin.

In this method III, the labeling materials used in labeling antibodies include those described in the above-mentioned method I and method II, and are used in the same way.

The amino acid sequences of the propeptides of osteocalcins and proosteocalcins are not always the same among a human being and animal species. Therefore, in the aforesaid method I, method II and method III and kids therefor of this invention, antibodies to be used are selected depending on a human being or animal species to be inspected. Namely, there are used antibodies specific against propetides of osteocalcins and proosteocalcins corresponding, respectively, to a human being and animal species to be inspected. specifically, for inspection samples of human beings are used antibodies against human propeptide or human proosteocalcin. Immunological measurement of this invention is advantageously

applied for human beings or animals (particularly, rats, mice or dogs).

According to researches of the present inventors, it was found that, in the immunological measurement methods of this invention, when animal serum, particularly equine serum is added to the standard substance, measurement sensitivity further increases. Further, it was also revealed that, in the immunological measurement methods of this invention, preferred results can be obtained by carrying out the immunological reaction at a temperature of normal temperature or less, preferably about 15°C or less, particularly about 10°C or less.

Hereafter, this invention is detailedly described by example, but no limited to them. Percents in examples mean weight %.

Example 1

Obtainment of an antibody:

(1) Preparation of an antigen

① Synthesis of the propeptide of human osteocalin

The propeptide of human osteocalcin, represented by the aforesaid formula [I], was synthesized. This synthesis was carried out using a peptide synthesis machine produced by ABI Co. The resultant peptide was named ProOst-26.

② Preparation of a linkage product between the antigen (ProOst-26) and a carrier protein

Keyhole limplet hemocyanin (KLH) was used as a carrier protein. A KLH-ProOst-26 linkage product was prepared using KLH, ProOst-26 and a water soluble carbodiimide. A product obtained by dialysis after reaction for 3 hours was used as an antigen.

(2) Preparation of an antibody

An emulsion containing the KLH-ProOst-26 linkage product was prepared using Freund's complete adjuvant, and a rabbit was immunized therewith every two weeks. The second or later immunization was carried out using Freund's incomplete adjuvant. Increase of the antibody titer was confirmed, and then exsanguination was carried out and antiserum was purified according to the method of Ey et al. (P.L. Ey et al., Immunochemistry, 15, 429, 436 (1978)). Namely, 5 ml of the antiserum was flowed into a protein A-Sephalose column (gel volume: 5 ml) equilibrated with 0.1 M phosphate buffer (pH 8.0), and after washing, IgG was purified from the column using 0.1 M sodium citrate buffer (pH 3.0) to obtain an anti-ProOst-26 antibody.

Example 2

Measurement of the propeptide of human osteocalcin by a sandwich method EIA:

(1) Preparation of beads having immobilized the antibody thereon

Polystyrene-made beads (diameter: 6 mm) having a surface like a mirror surface was, after sufficient washing, allowed to stand at a temperature of 4°C a whole day and night in a PBS solution having a concentration of 20 µg/ml of the anti-ProOst-26 antibody obtained in Example 1. Then, the resultant beads were washed, and allowed to stand at a temperature of 4°C a whole day and night in a PBS solution of 1% bovine serum albumin (BSA) to make post-coating treatment, whereby beads having immobilized thereon the anit-ProOst-26 antibody were obtained.

(2) Preparation of an HRP-labeled antibody

To 1 ml of a solution of 2.0 mg/ml the anti-ProOst-26 antibody in PBS were added 100 µl of 1M acetate buffer (pH 4.2) and a solution of 40 µg of pepsin in 20 µl of the same buffer, and the mixture was subjected to reaction at 37°C for 4 hours.

After completion of the reaction, separation was carried out using a Sephadex G25 column (ø 2cm x

45cm) equilibrated with PBS and thereby F(ab')$_2$ was recovered. To 2 ml of a solution of 1 mg/ml of F(ab')$_2$ in 0.01 M phosphoric acid - 0.15 M NaCl (pH 7.4) was added 50 µl of a solution of 10 mg/ml MBS in dimethylformamide, and reaction was carried out at a temperature of 25° C for 30 minutes. Then, gel filtration was carried out using a column packed with Sephadex G-25 and 0.1 M phosphate buffer (0.1 M PB) (pH 6.0) to separate the maleimdated antibody from unreacted MBS.

On the other hand, 120 µl of a solution of 60 mg/ml of S-acetylmercaptosuccinic anhydride in dimethylformamide was added to 2 ml of a solution of 10 mg/ml of HRP in 0.1 M PB (pH 6.5), and the mixture was subjected to reaction at 25° C for 2 hours.

Then, 800 µl of 0.1 M trishydrochloride buffer (pH 7.0), 160 µl of 0.1 M EDTA and 1.6 ml of 1M hydroxylamine were added, and the mixture was subjected to reaction at 0° C for 4 hours. Thereafter, the reaction solution was put in a collodion bag, and dialyzed against a 5 mM EDTA-containing 0.1 M PB (pH 6.0) solution at 4° C for 3 days to obtain thiolized HRP.

Then, 2 mg of the maleimidated antibody and 4 mg of the thiolized HRP were mixed, and the mixture was concentrated under ice cooling using a collodion bag until a protein concentration of 4 to 10 ng/ml is attained, and then allowed to stand at 15 to 20° C overnight. The resultant solution was subjected to gel filtration using a column packed with ultrogel AcA 44 (produced by LKB Co.) to obtain an HRP-labeled anti-ProOst-26 antibody F(ab')$_2$. By the same way, an HRP-labeled anti-ProOst-26 antibody IgG was also obtained.

(3) Sandwich EIA measurement system

In test tubes were put respective one bead having immobilized thereon the anti-ProOst-26 antibody F-(ab')$_2$, prepared in (1), respective 200 µl of 0.05M TBS (pH 8.0) containing 1% BSA and containing in the range of 0 to 20 ng/ml purified propeptide (standard substance) of human osteocalcin, and 200 µl portions of a solution of the HRP-labeled antibody prepared in (2) in 0.05 M TBS (pH 8.0) containing 1% BSA, and the mixture was subjected to incubation at 4° C for 16 hours. Then, after removal of the solution in each test tube by suction, the test tube was washed with 0.05 M TBS (pH 8.0). Then, in each test tube was put 0.4 ml of 0.1 M phosphate/citrate buffer (pH 4.3) containing 0.02% 3,3'5,5'-tetramethylbenzidine hydrochloride and 2.5 mM $H_2O_2$. Reaction was carried out at a temperature of 25° C for 30 minutes, and then enzyme reaction was stopped by addition of 1 ml of 1N aqueous sulfuric acid solution as a reaction discontinuation agent. The resultant solution was measured for absorption intensity at a wavelength of 450 nm using a spectrophotometer. The resultant absorption intensities were plotted against the standard substance concentrations of 0 to 20 ng/ml to obtain a calibration curve which was shown in Fig. 1. From the result, it is seen that, by using the measurement method of this invention, it is possible to measure the propeptide up to 0.05 ng/ml with good accuracy.

Further, measurement systems were constituted using the HRP-labeled anti-ProOst-26 antibody F(ab')$_2$ and the HRP-labeled anti-ProOst-26 antibody IgG, respectively, and the resultant respective absorption intensities were plotted against standard substance concentrations [0 to 6 ng/ml], whereby N/S ratios were calculated. The results were shown in Table 1.

Table 1

| Labeled antibody | $OD_{450}$ Propeptide 0 ng/ml | $OD_{450}$ Propeptide 6 ng/ml | N/S ratio (%) |
|---|---|---|---|
| IgG | 0.079 | 0.64 | 12.3 |
| F(ab')$_2$ | 0.042 | 0.60 | 7.0 |

Example 3

In measurement of the propeptide in patient specimens, a test of addition of a standard substance (synthesized propeptide) to sera and recovery thereof was carried out in order to examine whether the method of this invention is a quantitative measurement system or not. The results are shown in Table 2. As is apparent from the table, its recoveries were very large values of 400 to 800%. The reason is surmised to be that there is a difference between the structure of the synthesized propeptide, used as a standard substance, in the assay buffer and the actual structure of the propeptide in human serum, and at the same time that the propeptide is turned to a structure easy for the antibody to recognize by interaction with some components in human serum. Thus, serum not containing substances having cross reactivity was added to

the standard substance, and as a result the recoveries were turned to 80 to 120% and thus greatly improved. The results are shown in Table 3.

Table 2 (a)

Specimen 1 Normal adult

| Specimen dilution | specimen value (ng/ml) | Addition quantity (ng/ml) | Measured value (ng/ml) | Recovery quantity (ng/ml) | Recovery (%) |
|---|---|---|---|---|---|
| x 4 | 0.032 | 0.185 | 0.920 | 0.888 | 480 |
| | | 0.830 | 4.350 | 4.318 | 520 |
| x 8 | 0.026 | 0.185 | 1.020 | 0.994 | 537 |
| | | 0.830 | 6.000 | 5.974 | 720 |
| x 16 | 10.020 | 0.185 | 0.960 | 0.940 | 508 |
| | | 0.830 | 5.100 | 5.080 | 612 |

Table 2 (b)

Specimen 2 Normal young child

| Specimen dilution | specimen value (ng/ml) | Addition quantity (ng/ml) | Measured value (ng/ml) | Recovery quantity (ng/ml) | Recovery (%) |
|---|---|---|---|---|---|
| x 4 | 0.290 | 0.185 | 1.300 | 1.010 | 546 |
| | | 0.830 | 3.850 | 3.560 | 429 |
| x 8 | 0.150 | 0.185 | 1.220 | 1.070 | 578 |
| | | 0.830 | 4.900 | 4.750 | 572 |
| x 16 | 0.079 | 0.185 | 0.980 | 0.901 | 487 |
| | | 0.830 | 4.900 | 4.821 | 581 |

Table 3 (a)

Specimen 1 Normal adult

| Specimen dilution | specimen value (ng/ml) | Addition quantity (ng/ml) | Measured value (ng/ml) | Recovery quantity (ng/ml) | Recovery (%) |
|---|---|---|---|---|---|
| x 4 | 0.043 | 0.185 | 0.208 | 0.165 | 89 |
| | | 0.830 | 0.915 | 0.872 | 105 |
| x 8 | 0.020 | 0.185 | 0.229 | 0.209 | 113 |
| | | 0.830 | 0.784 | 0.764 | 92 |
| x 16 | 0.014 | 0.185 | 0.201 | 0.187 | 101 |
| | | 0.830 | 0.910 | 0.896 | 108 |

Table 3 (b)

Specimen 2 Normal young child

| Specimen dilution | specimen value (ng/ml) | Addition quantity (ng/ml) | Measured value (ng/ml) | Recovery quantity (ng/ml) | Recovery (%) |
|---|---|---|---|---|---|
| x 4 | 0.231 | 0.185 | 0.422 | 0.191 | 103 |
| | | 0.830 | 0.903 | 0.672 | 81 |
| x 8 | 0.118 | 0.185 | 0.272 | 0.154 | 83 |
| | | 0.830 | 1.081 | 0.963 | 116 |
| x 16 | 0.063 | 0.185 | 0.259 | 0.196 | 106 |
| | | 0.830 | 0.818 | 0.755 | 91 |

Example 4

Measurement of the propeptide of human osteocalcin in patient specimens:

Blood specimens of various patients (young children and adults) were measured by the measurement method of Example 2. The results were shown in Fig. 2. The measured values were high in the patients of infant osteogenesis imperfecta, hyperthyroidism and precocious puberty, and low in the patients of diabetes and chondrodystrophy and in part of the patients of cryptogenic low stature.

Specimens of high value were not recognized in normal adults.

Example 5

Immunological measurement by a sandwich method was carried out according to the method of Example 2 using an immobilized antibody obtained by immobilizing an antibody against the 20 residues at the N-terminus of human osteocalcin (Clon-12F) (refer to PCT/JP90/00155) and the HRP-labeled anti-OstPro-26 antibody ($F(ab')_2$) prepared in Example 2.

In this connection, as the standard substance was used a fused protein obtained from a transformant XLI-Blue F [pKOC28] prepared according to the method disclosed in Japanese Patent Application No.

159909/1990. A calibration curve obtained as a result is shown in Fig. 3.

It is revealed from Fig. 3 that a highly sensitive measurement system was obtained.

Example 6

Preparation of monoclonal antibodies

A peptide of the C-terminal 13 residues of the propeptide of human osteocalcin was synthesized, a KLH conjugated of this peptide was prepared, and a Balb/C mouse was immunized with this conjugate. According to the method of Keller and Milstein, hybridomas 3F9 and 4E12 were obtained as antibody-producing clones. Monoclonal antibodies produced by these hybridomas are named M-3F9 and M-4E12, respectively. Subclasses of these monoclonal antibodies were equally IgG1.

A measurement system was prepared according to the method disclosed in Example 2, using M-3F9 or M-4E12 as the solid phase antibody and using as the labeled antibody the polyclonal antibody obtained in (2) of Example 1. The propeptide of human osteocalcin was measured using this measurement system and in the same manner as in (3) of Example 2. The results are shown in Fig. 4. It is understood from Fig. 4 that the propeptide of human osteocalcin can be measured in high sensitivity also by the combination of the monoclonal antibody and the polyclonal antibody.

Example 7

Preparation of a polyclonal antibody

The C-terminal 13 residues (pC-13) of the propeptide of human osteocalcin was prepared, and then, a polyclonal antibody (apC-13) was obtained using this residue and according to the same manner as in Example 1.

Preparation of a measurement system

The following two measurement systems were prepared in the same way as in Example 2.

| | Solid phase antibody | Labeled antibody |
|---|---|---|
| (i) | ap-26 | ap-26 |
| (ii) | apC-13 | ap-26 |

As ap-26 antibody was used one of Example 1

The propeptide of human osteocalcin was measured in the same way as disclosed in (3) of Example 2, using these measurement systems. The results are shown in Fig. 5. It is also seen from this Fig. 5 that the propeptide of human osteocalcin can be measured in high sensitivity according to this invention.

Example 8

Antibody (ap-26) obtained in (2) of Example 1 was radiolabeled with $I^{125}$. Its radioactivity was 1,530,000 cpm/$\mu$g. Respective 10,000 cpm of the labeled antibody and the propeptide of human osteocalcin or proosteocalcin in respective concentrations of 0.012, 0.05, 0.2, 0.8 and 3.2 ng/ml were put into test tubes, respectively. The mixtures were incubated at 37°C for 1 hour. Thereafter, the resultant reaction solutions were reacted with solid phases having immobilized antibody ap-26 thereon (37°C, 1 hour), respectively. After washing the resultant solid phase three times with PBS were counted the quantities of respective $I^{125}$-labeled ap-26 linked to the respective solid phase ap-26. The results are shown in Fig. 6.

In Fig. 6, the concentration of proosteocalcin is a value turned into the quantity of the propeptide. The axis of ordinate in Fig. 6 is B/Bo (Bo is the total quantity of $I^{125}$-labeled ap-26 added; and B is the quantity of $I^{125}$-labeled ap-26 linked to the solid phase when the propeptide or proosteocalcin was added). From this Fig. 6, it is seen that the propeptide and proosteocalcin are equally recognized.

Example 9

As a solid phase antibody was used one wherein antibody ap-26 was immobilized. In test tubes were

put, respectively, respective one bead having immobilized thereon antibody ap-26 prepared in Example 3 (B)(1), 200 µl portions of 1% BSA-containing 0.05 M TBS (pH 8.0) containing purified human osteocalcin in the range of 0 to 20 ng/ml, and 200 µl portions of a solution in 1% BSA-containing 0.05 M TBS (pH 8.0) of either an antibody obtained by labeling with HRP the polyclonal antibody (N 20) disclosed in Example 1 of the PCT application (PCT/JP90/00155) or an antibody obtained by labeling with HRP the polyclonal antibody (C7) disclosed in Example 2, and the mixtures were subjected to incubation at 4° C for 16 hours.

Then, after removal of the solution in each test tube by suction, the test tube was washed with 0.05 M TBS (pH 8.0). Then, in each test tube was put 0.4 ml of 0.1 M phosphate/citrate buffer (pH 4.3) containing 0.02% 3,3',5,5'-tetramethylbenzidine hydrochloride and 2.5 mM $H_2O_2$. Reaction was carried out at a temperature of 37° C for 30 minutes, and then enzyme reaction was stopped by addition of 1 ml of 1N aqueous sulfuric acid solution as a reaction discontinuation agent. The resultant solution was measured for absorption intensity at a wavelength of 450 nm using a spectrophotometer. The results are shown in Fig. 7.

The results in Fig. 7 shows that proosteocalcin can be measured in high sensitivity by this invention. From the results in Fig. 7, it is seen that the combina tion of the antibodies ap-26 and aOC-C7 gives better sensitivity, compared with the combination of the anti-bodies ap-26 and aOC-N20.

Example 10 (Investigation of specificity of measurement systems)

(a) Specificity to the propeptide of human osteocalcin
(b) Specificity to the proosteocalcin of human osteocalcin

According to the methods of the propeptide measurement system of Example 2 and the proosteocalcin measurement system of Example 9, specificity of each measurement system was investigated. The results were shown in Fig. 8. Namely in (a), it was revealed that there was specificity to the propeptide in measurement in the range of the propeptide of human osteocalcin of 0 to 50,000 ng/ml and proosteocalcin of 0 to 50 ng/ml. On the other hand, in (b), it was revealed that, measurement in the range of the propeptide of human osteocalcin of 0 to 50,000 ng/ml and proosteocalcin of 0 to 5 ng/ml, dose dependance was only observed on proosteocalcin and thus there was specificity to proosteocalcin.

Example 11 (Monitering the secretion of human osteocalcin from human osteoblasts)

Culture was carried out in 96 holes using the osteoblasts of Koshihara et al. [BBRC, 145, 651-657 (1987)], and the changes with time lapse were investigated of secretion (24 days) of the propeptide (P-OC) of human osteocalcin and complete human osteocalcin (I-OC) in the culture supernatants. Culture was carried out in the presence of $10^{-9}$ M 1.25 $(OH)_2D_3$. The results were shown in Fig. 9. It was revealed from Fig. 9 that although the secretion level of propeptide (P-OC) was almost above a certain value, complete human osteocalcin (I-OC) disappeared, and therefore, the propeptide finely reflects the function of the osteoblasts.

On the other hand, from the start of the culture was monitored accumulation of calcium (Ca), phosphorus (P) and complete human osteocalin in the cell layer, and the results were shown in Fig. 10. This Fig. 10 shows rapid increase of complete human osteocalcin by the 11th day after the start of the culture. This clearly indicates that the decrease of complete huamn osteocalcin in Fig. 9 is due to the accumulation thereof in the cell layer.

It is seen, from these things, that the propeptide has a possibility to reflect the activity of the osteoblasts more effectively than human osteocalcin.

Example 12

Before and after administration of growth hormone (GH) to young children defective in growth hormone was measured the propeptide (P-OC) of human osteocalcin in the blood of the children. The measurement was carried out according to the method of Example 2. The results were shown in Fig. 11 and Fig. 12. Fig. 11 shows the results before the administration of growth hormone (GH) and indicates that the quantity of the propeptide has a good correlation with increase (cm/year) of the hight of the young children.

On the other hand, the ratio of the quantity of the propeptide after the administration of growth hormone (GH) (the propeptide before the administration/the propeptide after the administration) was plotted in the axis of ordinate, and the ratio of increase of the hight (increase before the administration/increase after the administration) was plotted in the axis of abscissa. Fig. 12 as the result indicates that they exhibit a positive correlation.

It is seen from this that the rate of increase of the propeptide after administration of growth hormone

positively correlates with the rate of increase of the stature after the administration. It was revealed from these results that the method of this invention for measurement of the propeptide can provide a method for measurement of a marker capable of clearly indicating the state of bone formation in young children.

Example 13 (Monitoring of the metastasis of the cancer to the bones)

Two patients (a and b) having a cancer metastasized to the bones were treated with a carcinostatic, and after the start of the treatment, with lapse of the treatment, the values of the propeptide (P-OC) of human osteocalcin in the sera of these patients were measured by the method of Example 2. The results were shown in Fig. 13. As is apparent from Fig. 13, increase of proosteocalcin was observed after the start of the treatment both in (a) and (b). Although the reason of increase of proosteocalcin in these cases is not clear, it is surmised that probably, by the treatment of the metastasis to the bones, invasion of the cancer cells on the osteoblasts near the bones was prevented by the carcinostatic treatment, and as a result, the function of the osteoblasts was recovered and thereby production of the propeptide in the blood increased. From this, it was shown that the method of this invention is useful for judgment of the effect of treatment of the metastasis to bones by carcinostatics.

Example 14 (Measurement of the propeptide of the osteocalcin of a rat)

There was synthesized the propeptide of the osteocalcin of a rat having the following amino acid sequence (The EMBO Journal Vol 5. No. 8, 1885-1890, 1986).

$H_2N$-Lys-Pro-Ser-Asp-Ser-Glu-Ser-Asp-Lys-Ala-
Phe-Met-Ser-Lys-Gln-Glu-Gly-Ser-Lys-Val-Val-
Asn-Arg-Leu-Arg-Arg-COOH

A rabbit was immunized with this propeptide in the same way as in Example 1 (2) to obtain a polyclonal antibody against this propeptide. A sandwich measurement system was prepared according to the same method as in Example 2 using this polyclonal antibody. Using this measurement system was prepared a standard curve of the propeptide of rat osteocalcin. The result was shown in Fig. 14. From this Fig. 14, it was revealed that the propeptide of rat osteocalcin can also be measured by this invention.

Effect of the Invention

According to the immunological measurement method of this invention, the quantity of the propeptide of osteocalcin or proosteocalcin existing, respectively, in a very small quantity in an inspection sample, or the total quantity of them can be measured with distinction between them, in high sensitivity and accurately. The results of these measurements can be utilized extremely usefully in various clinical medicines. Since the results of these measurements generally reflect the states of osteogenesis, for example in case of human clinical medicines, they can be utilized as an indicator of osteogenesis in young children, etc. or for adult D treatment, treatment of metastases to borne, measurement of an ability to activate osteoblasts, judgment of the effect of treatment of the metastases of cancers to bones, etc.

Particularly, results of measurement of the propeptide of osteocalcin can extremely advantageously be utilized for diagnosis of the state of osteogenesis in bone metabolism. This means to measure the propeptide in high sensitivity and accurately was provided for the first time by this invention.

**Claims**

1. A method for immunologically measuring the propeptide of osteocalcin in an inspection sample of a sandwich method, wherein antibodies specifically recognizing the propeptide of osteocalcin are used as the antibody linked to the insoluble carrier (the first antibody) and the labeled antibody (the second antibody).

2. The method of claim 1 wherein both of said first antibody and said second antibody are polyclonal antibodies specifically recognizing the propeptide of osteocalcin.

3. The method of claim 1 wherein any one of said first antibody and said second antibody is a polyclonal antibody specifically recognizing the propeptide of osteocalcin, and the other is a polyclonal antibody or monoclonal antibody specifically recognizing the sequence of from the 14-position to the 26-position in the amino acid sequence of the propeptide of osteocalcin.

4. The method of any of claims 1 to 3 wherein said propeptide of osteocalcin is the propeptide of human osteocalcin, and the propeptide of human osteocalcin in a human inspection sample is measured.

5. The method of any of claims 1 to 3 wherein said propeptide of osteocalcin is the propeptide of osteocalcin of a specific animal, and the propeptide of osteocalcin in an inspection sample of the specific animal is measured.

6. The method of claim 5 wherein said specific animal is a rat, mouse or dog.

7. A kit for measuring the propeptide of osteocalcin in an inspection sample, comprising a combination of
(a) a solid phase antibody (the first antibody),
(b) a labeled antibody (the second antibody),
(c) a solubilizing agent,
(d) a washing agent
(e) a standard substance and
(f) when a labeled antibody labeled with an enzyme is used, a substrate for measuring enzyme activity and a reaction discontinuation agent,
in which kit for immunologically measuring the propeptide of osteocalcin, the first antibody and the second antibody are antibodies specifically recognizing the propeptide of osteocalcin.

8. The kit of claim 7 wherein both of the first antibody and the second antibody are polyclonal antibodies specifically recognizing the propeptide of osteocalcin.

9. The kit of claim 7 wherein any one of said first antibody and said second antibody is a polyclonal antibody specifically recognizing the propeptide of osteocalcin, and the other is a polyclonal antibody or monoclonal antibody specifically recognizing the sequence of from the 14-position to the 26-position in the amino acid sequence of the propeptide of osteocalcin.

10. The kit of claim 7 wherein said standard substance (e) contains animal serum.

11. A method for immunologically measuring proosteocalcin in an inspection sample by a sandwich method, wherein any one of the antibody linked to the insoluble carrier (the first antibody) and the labeled antibody (the second antibody) is an antibody specifically recognizing the propeptide of osteocalcin, and the other is an antibody specifically recognizing osteocalcin.

12. The method of claim 11 wherein said antibody specifically recognizing the propeptide of osteocalcin is a polyclonal antibody.

13. The method of claim 11 or 12 wherein said antibody specifically recognizing osteocalcin is a polyclonal antibody.

14. The method of claim 11 to 13 wherein said antibody specifically recognizing osteocalcin is a polyclonal antibody or monoclonal antibody specifically recognizing the region of the sequence of 14 amino acids at the C-terminus of osteocalcin.

15. The method of any of claims 11 to 14 wherein said proosteocalcin is human osteocalcin, and human proosteocalcin in a human inspection sample is measured.

16. The method of any of claims 11 to 14 wherein said proosteocalcin is the proosteocalcin of a specific animal, and the proosteocalcin in an inspection sample of the specific animal is measured.

17. The method of claim 16 wherein said specific animal is a rat, mouse or dog.

**18.** A kit for measuring proosteocalcin in an inspection sample, comprising a combination of
(a) a solid phase antibody (the first antibody),
(b) a labeled antibody (the second antibody),
(c) a solubilizing agent,
(d) a washing agent
(e) a standard substance and
(f) when a labeled antibody labeled with an enzyme is used, a substrate for measuring enzyme activity and a reaction discontinuation agent,
in which kit for immunologically measuring proosteocalcin, any one of the first antibody and the second antibody is an antibody specifically recognizing the propeptide of osteocalcin, and the other is antibody specifically recognizing osteocalcin.

**19.** The kit of claim 18 wherein said antibody specifically recognizing the propeptide of osteocalcin is a polyclonal antibody.

**20.** The kit of claim 18 or 19 wherein said antibody specifically recognizing osteocalcin is a polyclonal antibody.

**21.** The kit of any of claims 18 to 20 wherein said antibody specifically recognizing osteocalcin is a polyclonal antibody or monoclonal antibody specifically recognizing the region of the sequence of 14 amino acid at the C-terminus of osteocalcin.

**22.** The kit of claim 18 wherein said standard substance (e) contains animal serum.

**23.** A method for immunologically measuring the total quantity of the propeptide of osteocalcin and proosteocalcin in an inspection sample by a competition method, wherein as the antibody to be linked to the insoluble carrier or the insolubilizable antibody is used an antibody specifically recognizing the propeptide of osteocalcin.

**24.** The method of claim 23 wherein said antibody specifically recognizing the propeptide of osteocalcin is a polyclonal antibody.

**25.** The method of claim 23 wherein said antibody specifically recognizing the propeptide of osteocalcin is polyclonal antibody specifically recognizing the 1-position to 13-position in the amino acid sequence of the propeptide.

**26.** The method of any of claims 23 to 25 wherein is measured the total quantity of the propeptide of human osteocalcin and human proosteocalcin in a human inspection sample.

**27.** The method of any of claims 23 to 25 wherein is measured the total quantity of the propeptide of the osteocalcin of a specific animal in an inspection sample of the specific animal.

**28.** The method of claim 27 wherein said specific animal is a rat, mouse or dog.

**29.** A method for diagnosing the state of osteogenesis in the inspected human being or animal, based on the value of the propeptide of osteocalcin, proosteocalcin, or the total quantity of them measured by the method of claim 1, 11 or 23.

**30.** A polyclonal antibody specifically recognizing the propeptide of human osteocalcin.

**31.** A polyclonal antibody specifically recognizing the 14-position to 26-position in the amino acid sequence of the propeptide of human osteocalcin.

**32.** A polyclonal antibody specifically recognizing the 1-position to 13-position in the amino acid sequence of the propeptide of human osteocalcin.

**33.** A monoclonal antibody specifically recognizing the 14-position to 26-position in the amino acid sequence of the propeptide of human osteocalcin.

**34.** The monoclonal antibody of claim 33 wherein said monoclonal antibody is one produced by hybridoma 3F9 or 4E12.

Fig. 1

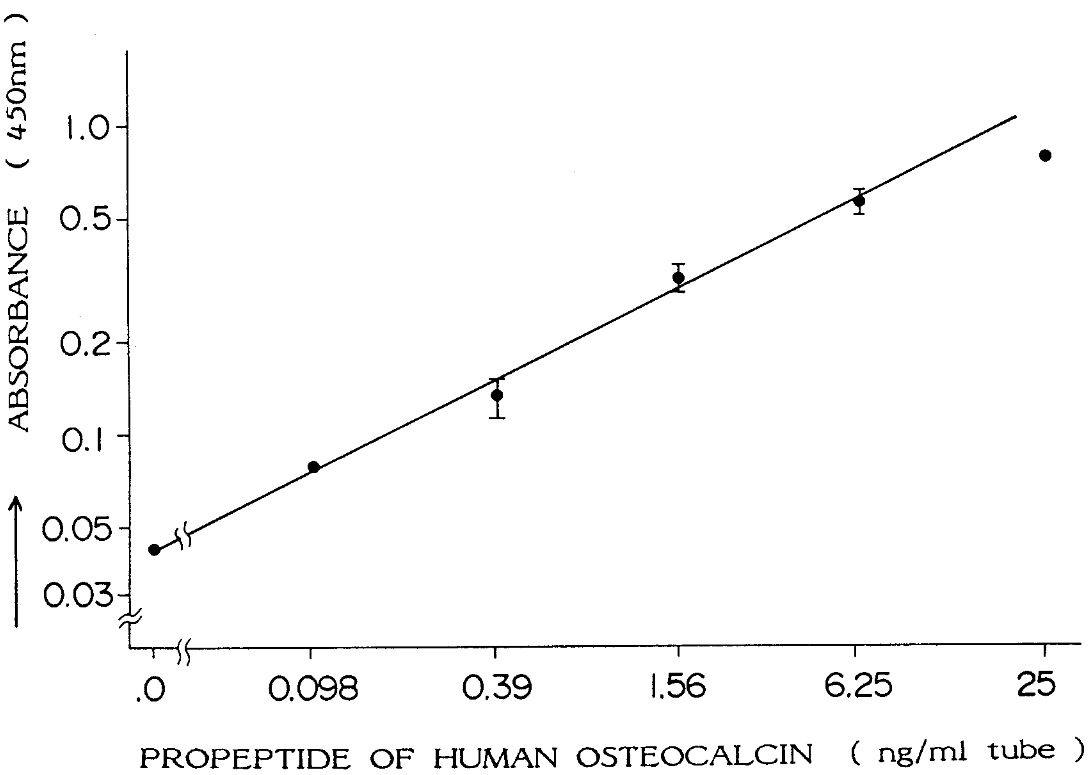
ABSORBANCE ( 450nm )
1.0
0.5
0.2
0.1
0.05
0.03
.0
0.098
0.39
1.56
6.25
25
PROPEPTIDE OF HUMAN OSTEOCALCIN ( ng/ml tube )

Fig. 2

PROPEPTIDE OF HUMAN OSTEOCALCIN ( ng/ml tube )

Number in ( ) : Age

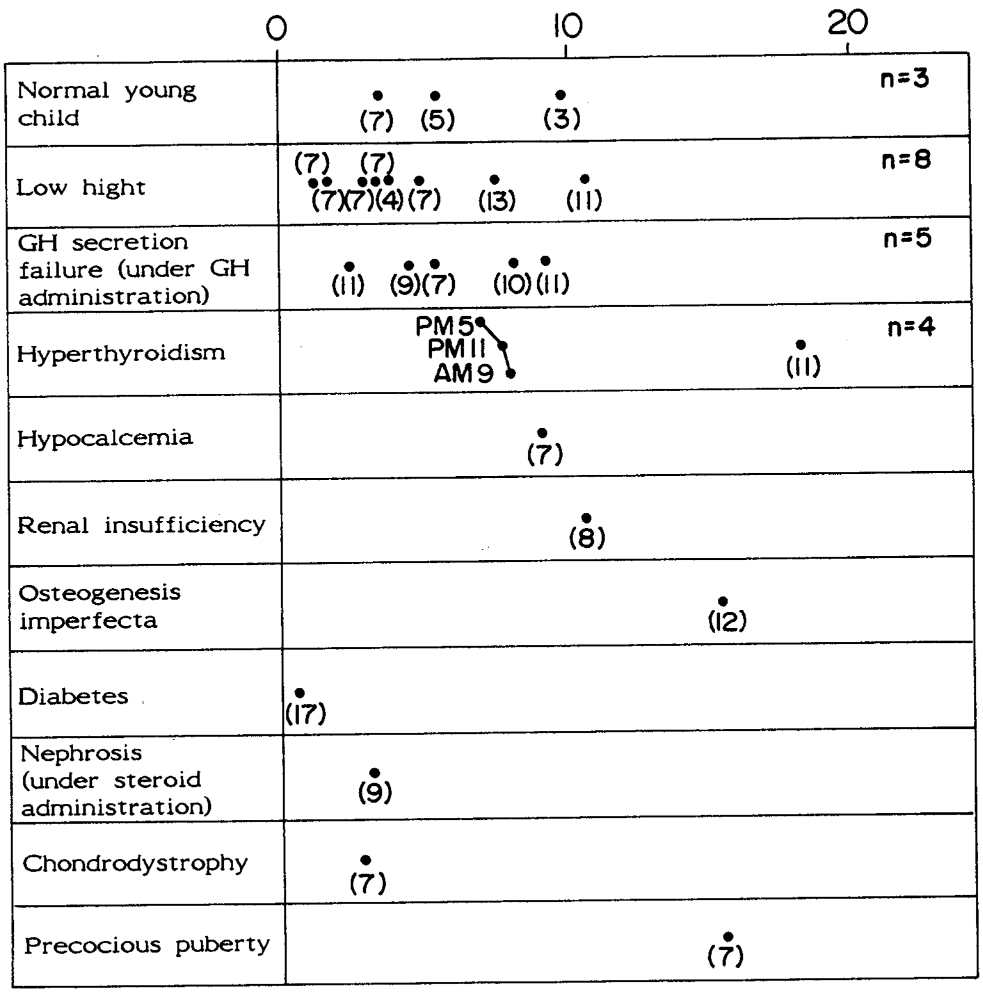

Fig. 3

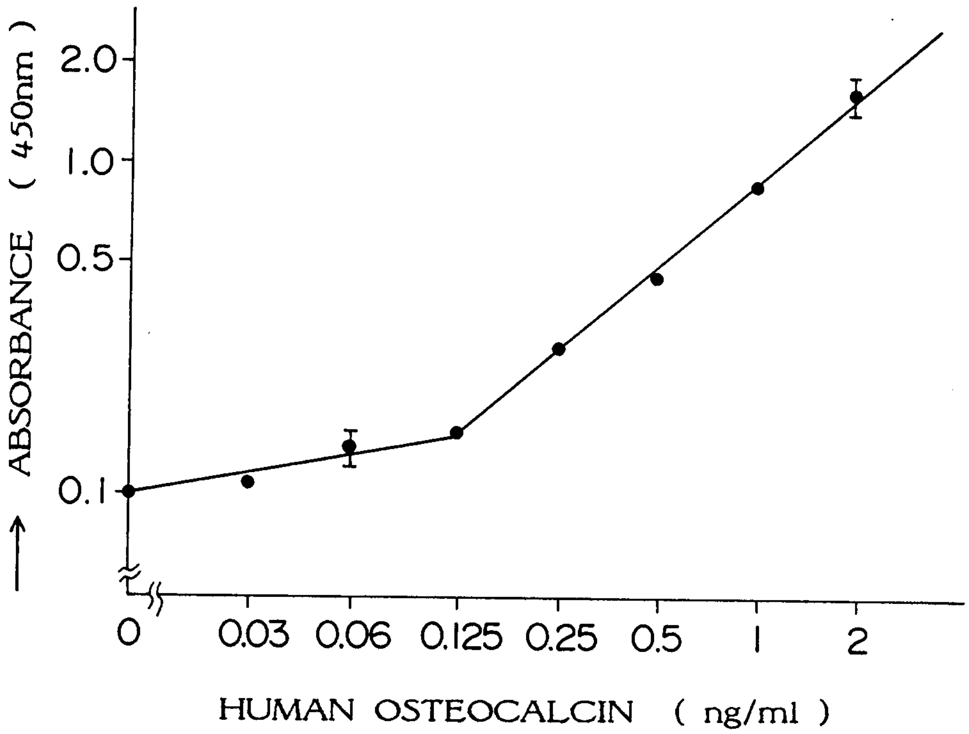
ABSORBANCE ( 450nm )
2.0
1.0
0.5
0.1
0
0.03
0.06
0.125
0.25
0.5
1
2
HUMAN OSTEOCALCIN ( ng/ml )

Fig. 4

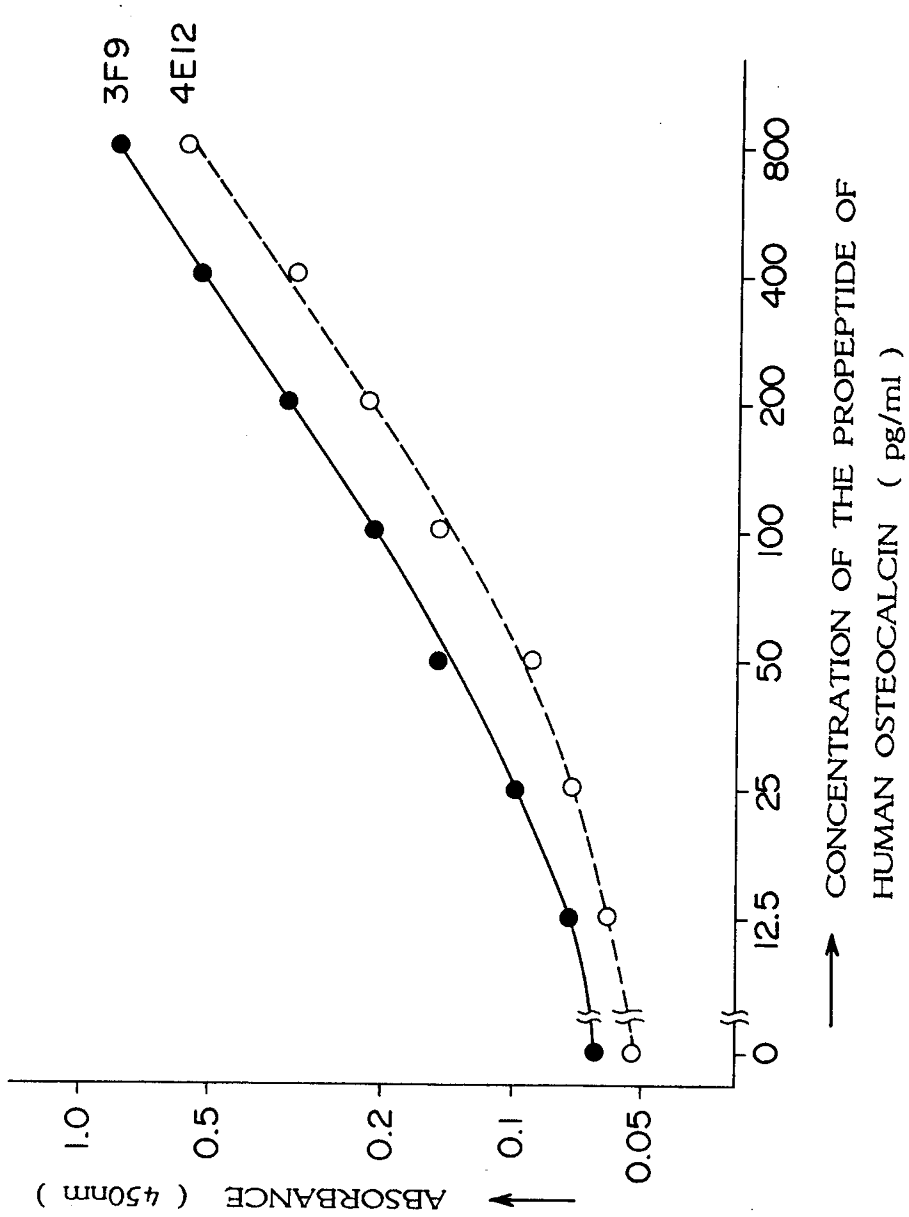
3F9
4E12
ABSORBANCE ( 450nm )
1.0
0.5
0.2
0.1
0.05
0
12.5
25
50
100
200
400
800
CONCENTRATION OF THE PROPEPTIDE OF
HUMAN OSTEOCALCIN ( pg/ml )

Fig. 5

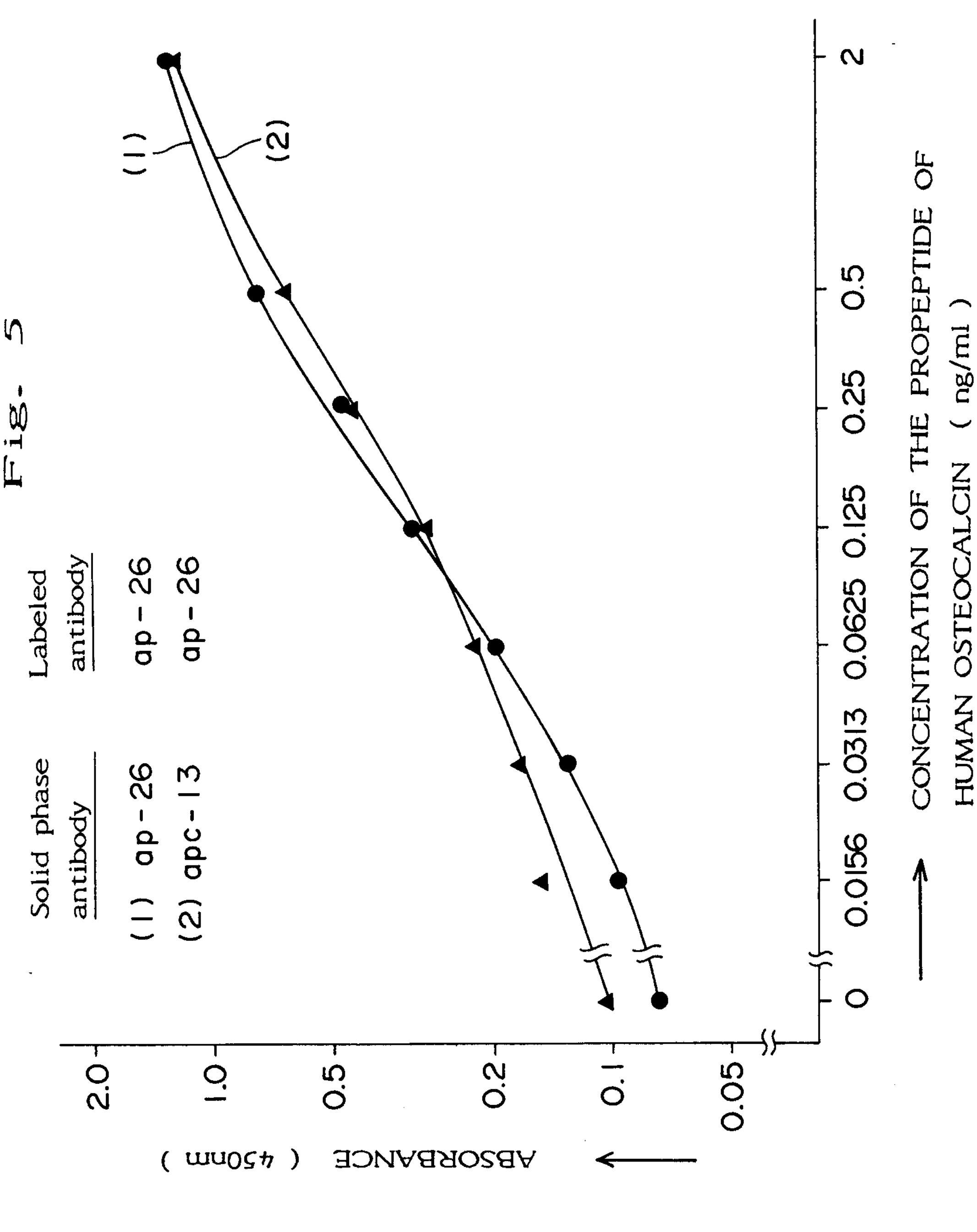
Solid phase antibody
Labeled antibody
(1) ap-26 ap-26
(2) apc-13 ap-26
(1)
(2)
ABSORBANCE ( 450nm )
2.0
1.0
0.5
0.2
0.1
0.05
0
0.0156
0.0313
0.0625
0.125
0.25
0.5
2
CONCENTRATION OF THE PROPEPTIDE OF HUMAN OSTEOCALCIN ( ng/ml )

Fig. 6

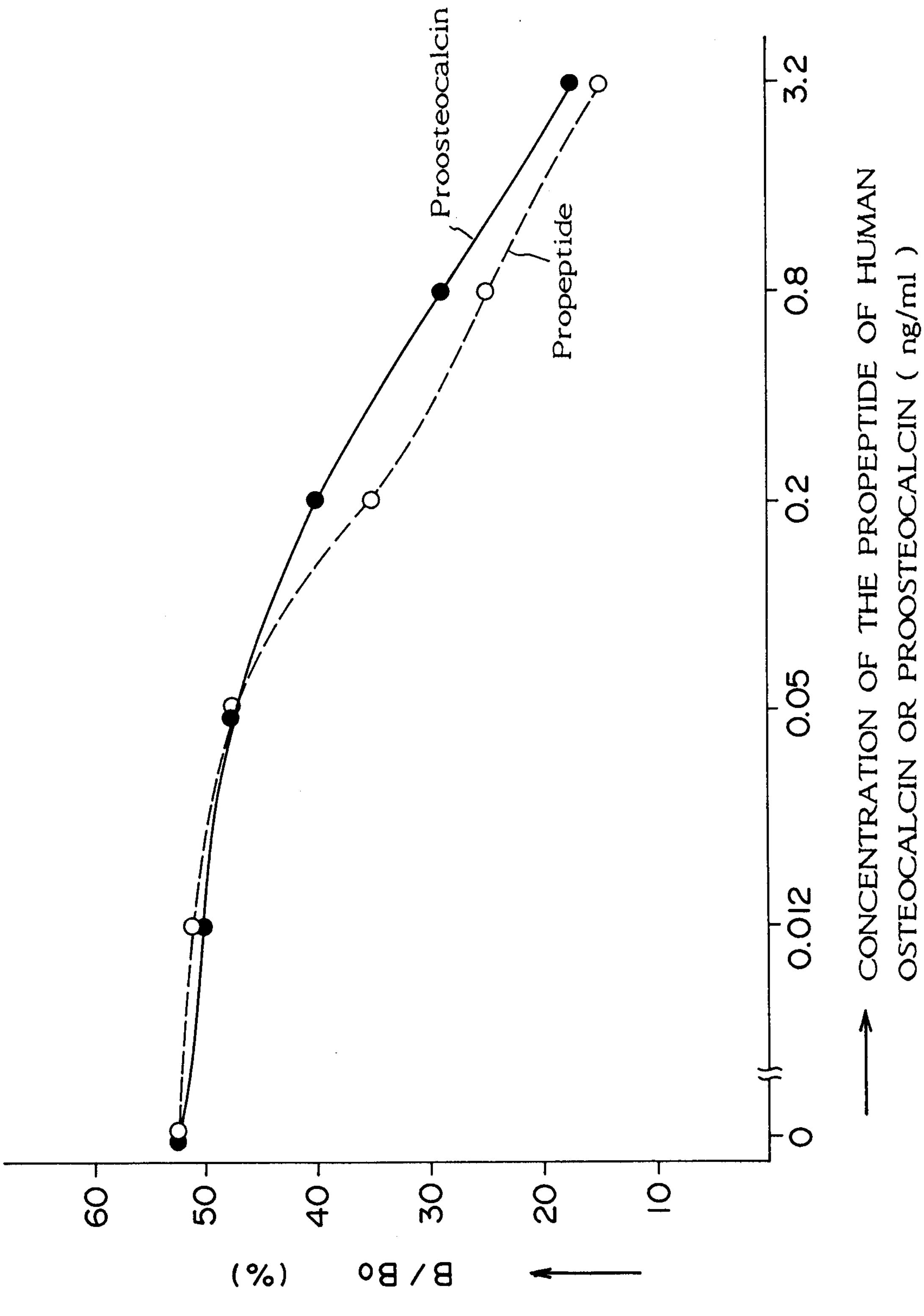
Proosteocalcin
Propeptide
60
50
40
30
20
10
B / Bo (%)
0
0.012
0.05
0.2
0.8
3.2
CONCENTRATION OF THE PROPEPTIDE OF HUMAN OSTEOCALCIN OR PROOSTEOCALCIN ( ng/ml )

Fig. 7

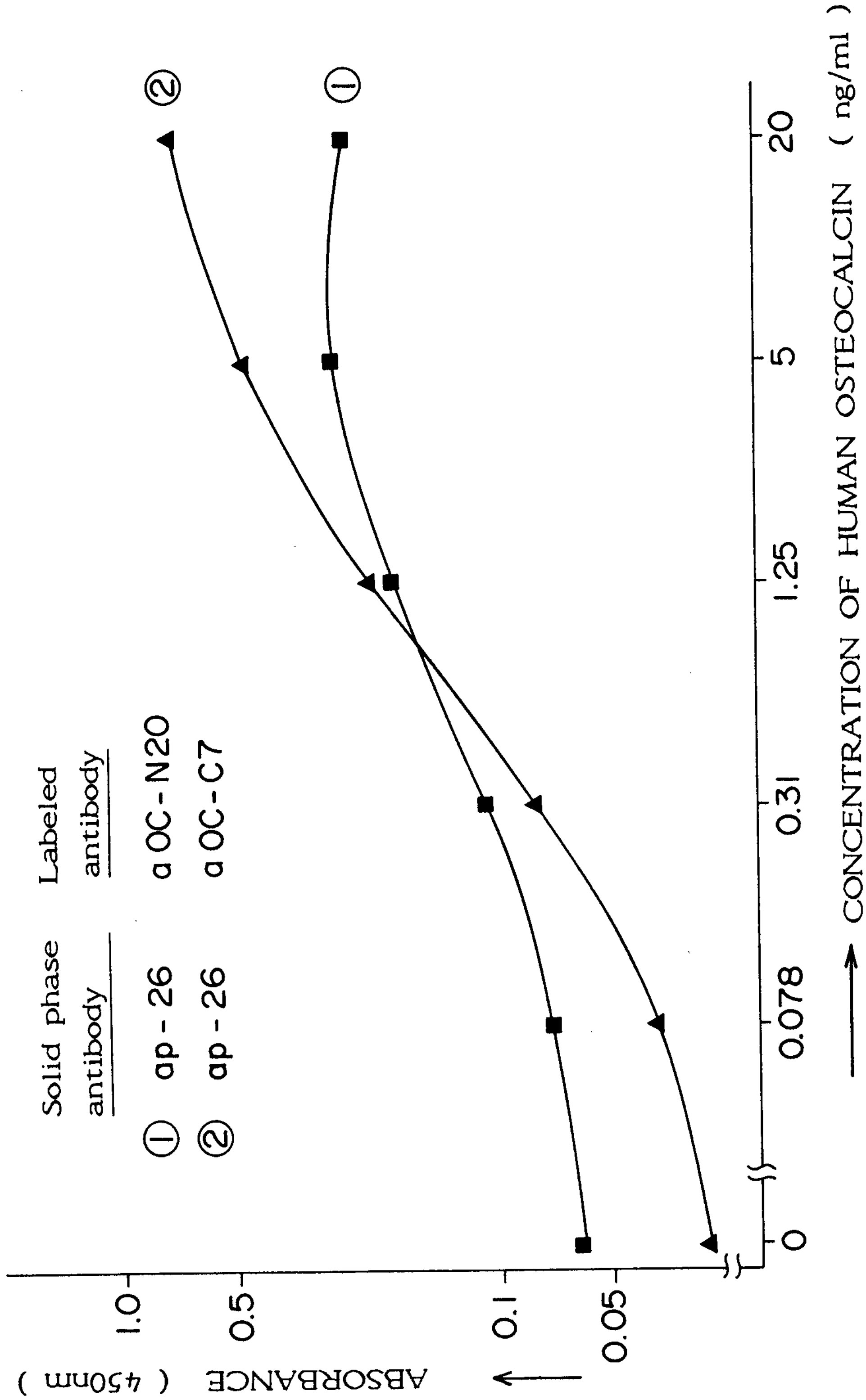
Solid phase antibody
Labeled antibody
① ap-26 α OC-N20
② ap-26 α OC-C7
ABSORBANCE ( 450nm )
1.0
0.5
0.1
0.05
0
0.078
0.31
1.25
5
20
CONCENTRATION OF HUMAN OSTEOCALCIN ( ng/ml )
①
②

Fig. 8

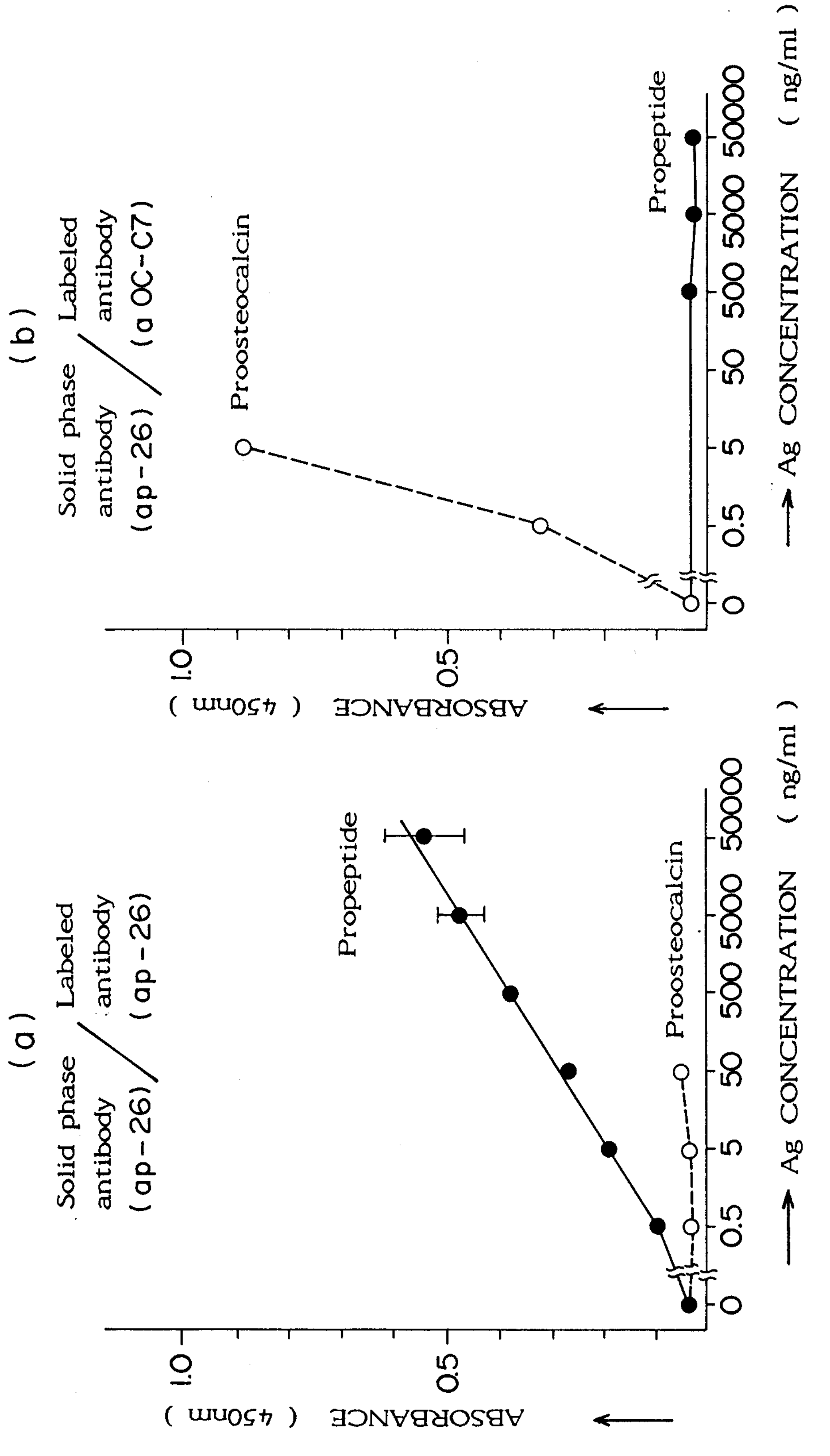
(a)
Solid phase antibody (ap-26) / Labeled antibody (ap-26)
Propeptide
Proosteocalcin
ABSORBANCE (450nm)
1.0
0.5
0
0.5
5
50
500
5000
50000
Ag CONCENTRATION (ng/ml)
(b)
Solid phase antibody (ap-26) / Labeled antibody (a OC-C7)
Proosteocalcin
Propeptide
ABSORBANCE (450nm)
1.0
0.5
0
0.5
5
50
500
5000
50000
Ag CONCENTRATION (ng/ml)

Fig. 9

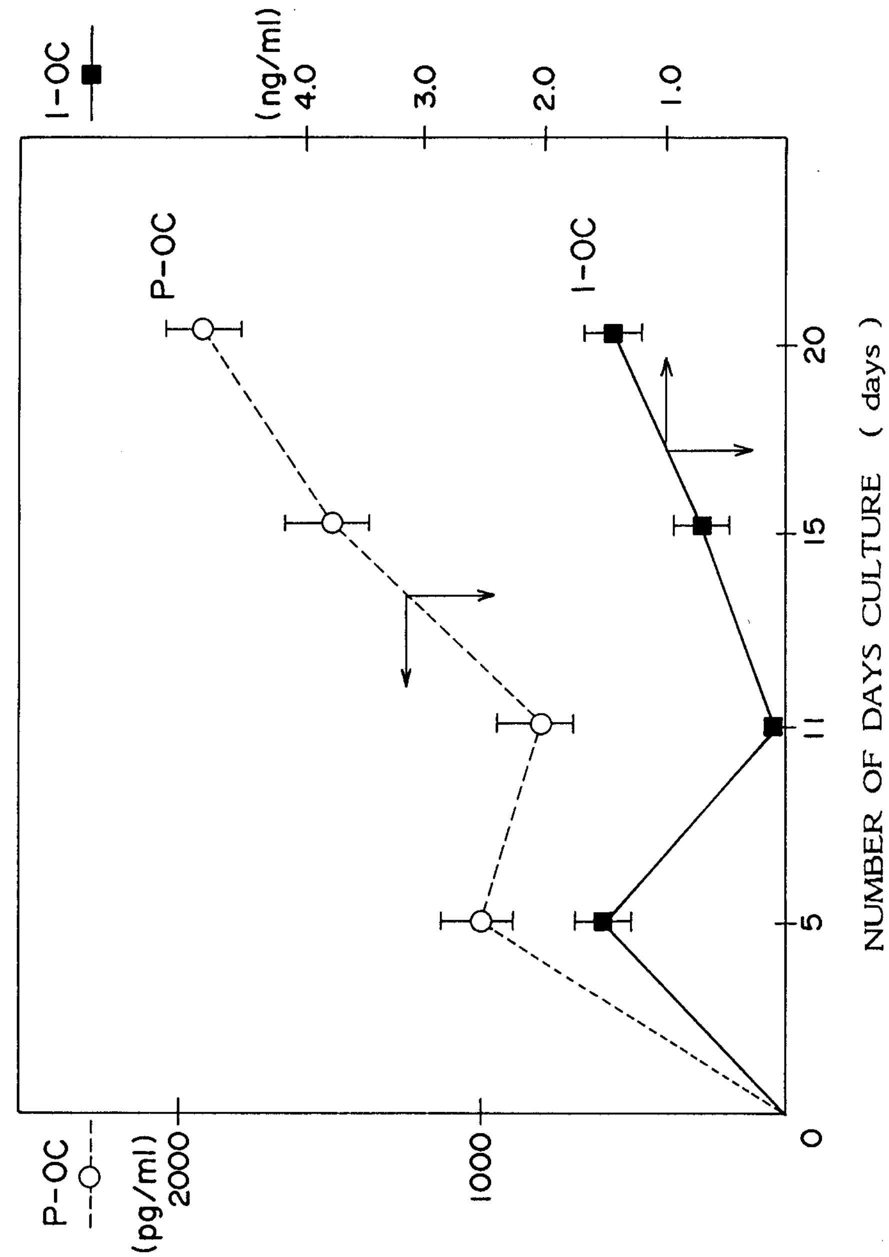
P-OC
(pg/ml)
2000
1000
I-OC
(ng/ml)
4.0
3.0
2.0
1.0
P-OC
I-OC
0
5
11
15
20
NUMBER OF DAYS CULTURE ( days )

Fig. 10

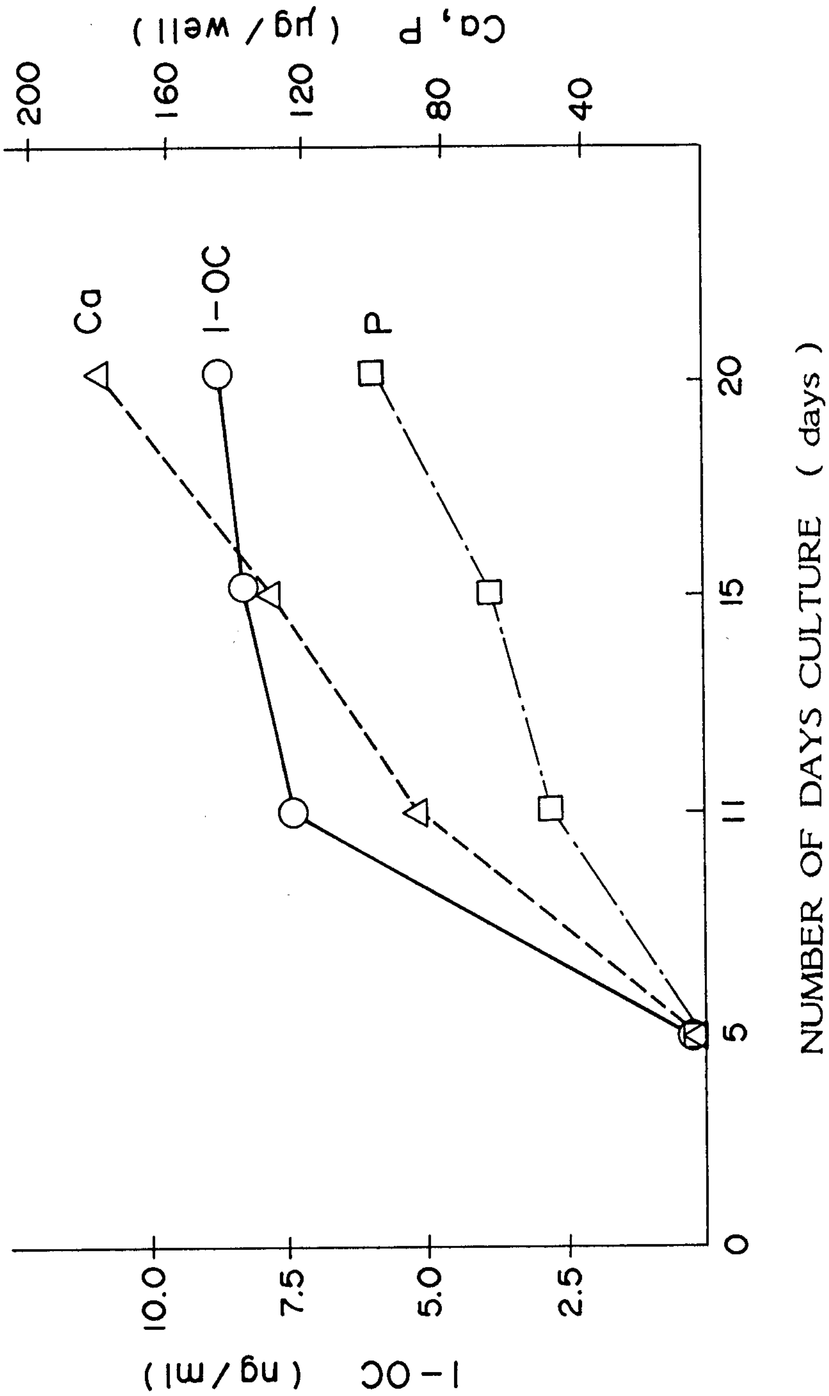
Ca
I-OC
P
I-OC (ng/ml)
10.0
7.5
5.0
2.5
Ca, P (μg/well)
200
160
120
80
40
0
5
11
15
20
NUMBER OF DAYS CULTURE (days)

Fig. 11

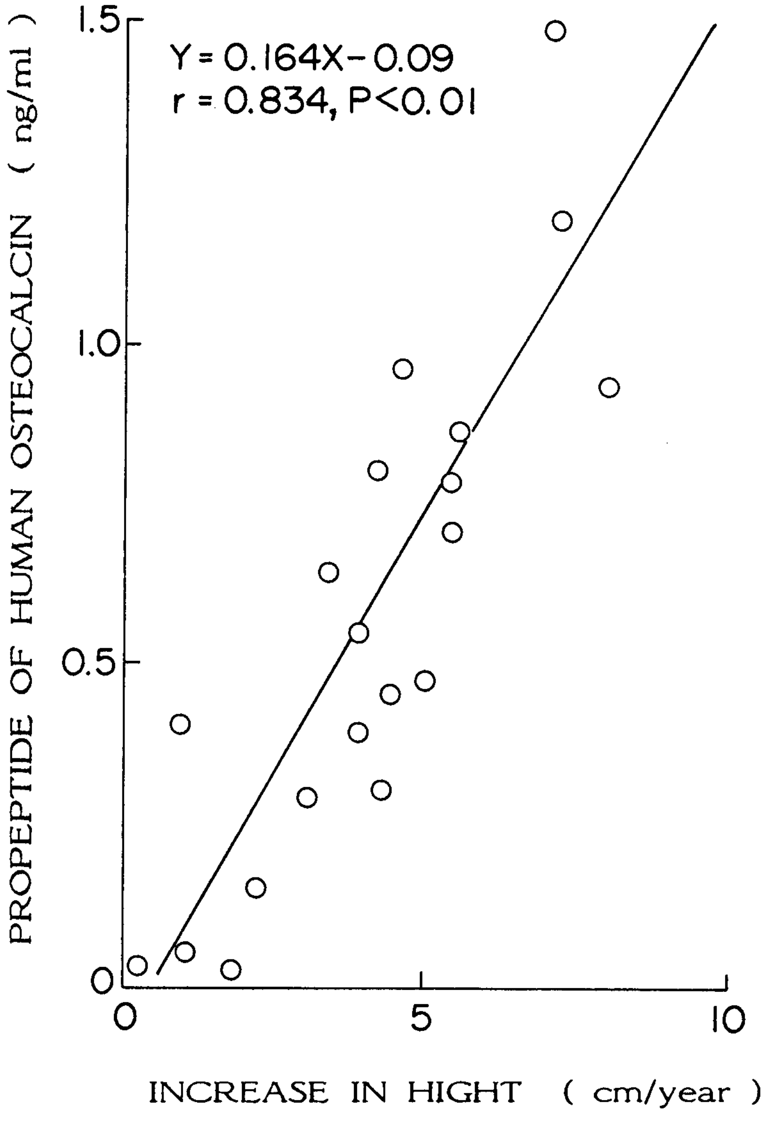
Y = 0.164X − 0.09
r = 0.834, P<0.01
PROPEPTIDE OF HUMAN OSTEOCALCIN ( ng/ml )
1.5
1.0
0.5
0
0
5
10
INCREASE IN HIGHT ( cm/year )

Fig. 12

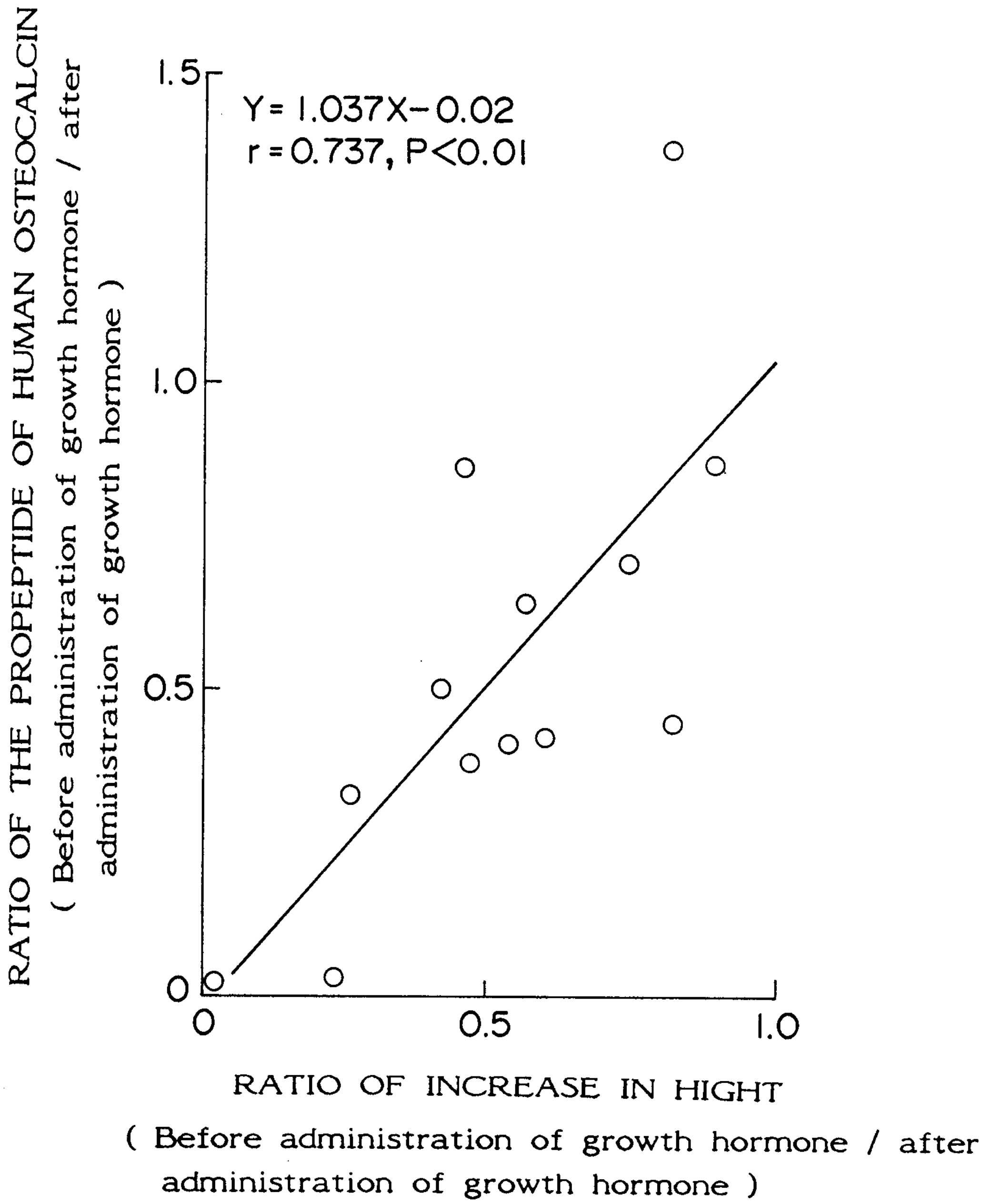
RATIO OF THE PROPEPTIDE OF HUMAN OSTEOCALCIN
( Before administration of growth hormone / after administration of growth hormone )
1.5
1.0
0.5
0
Y= 1.037X-0.02
r=0.737, P<0.01
0
0.5
1.0
RATIO OF INCREASE IN HIGHT
( Before administration of growth hormone / after administration of growth hormone )

Fig. 13

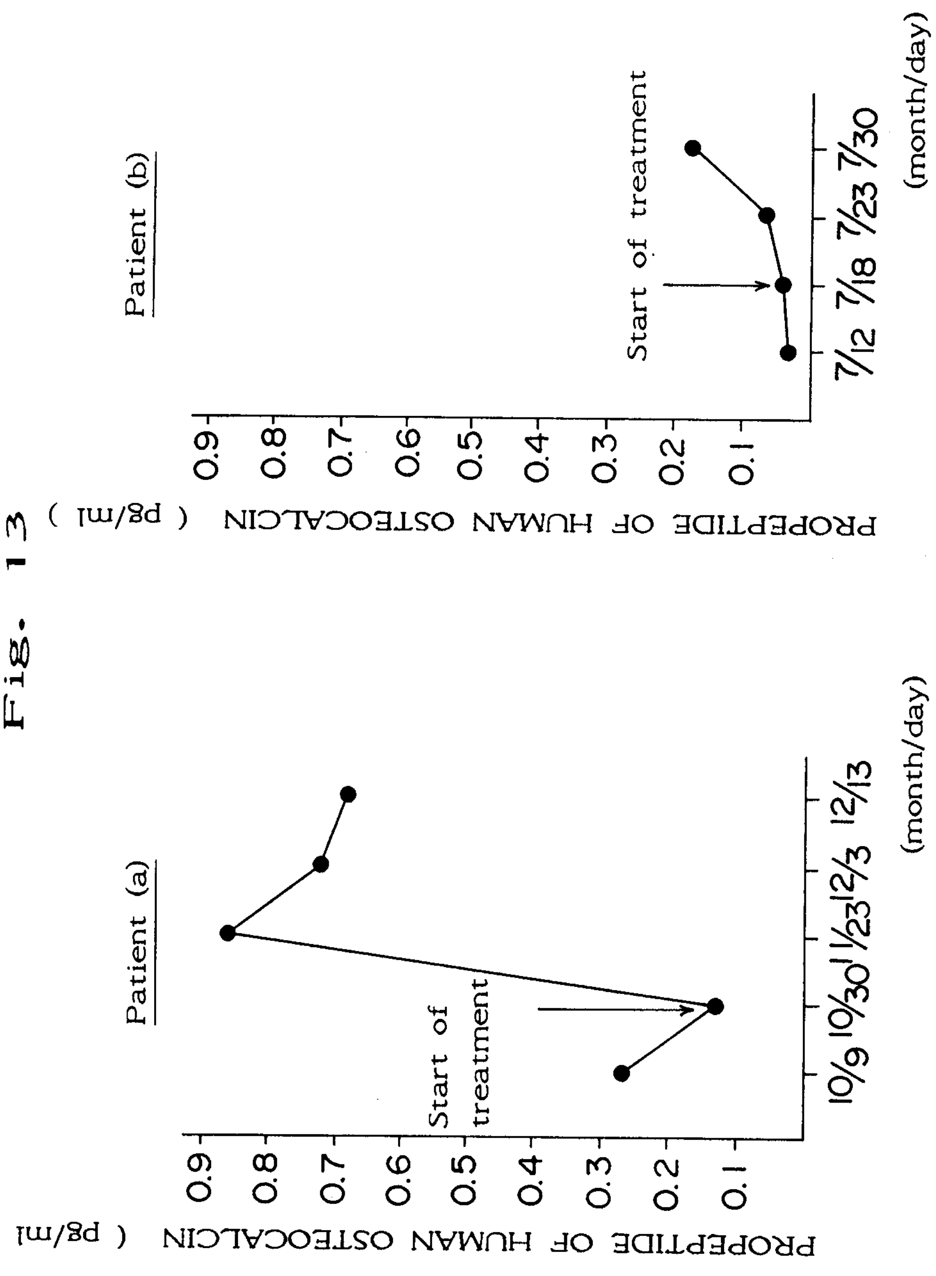
Patient (a)
Start of treatment
PROPEPTIDE OF HUMAN OSTEOCALCIN ( pg/ml )
0.9
0.8
0.7
0.6
0.5
0.4
0.3
0.2
0.1
10/9 10/30 11/23 12/3 12/13
(month/day)
Patient (b)
Start of treatment
7/12 7/18 7/23 7/30
(month/day)

Fig. 14

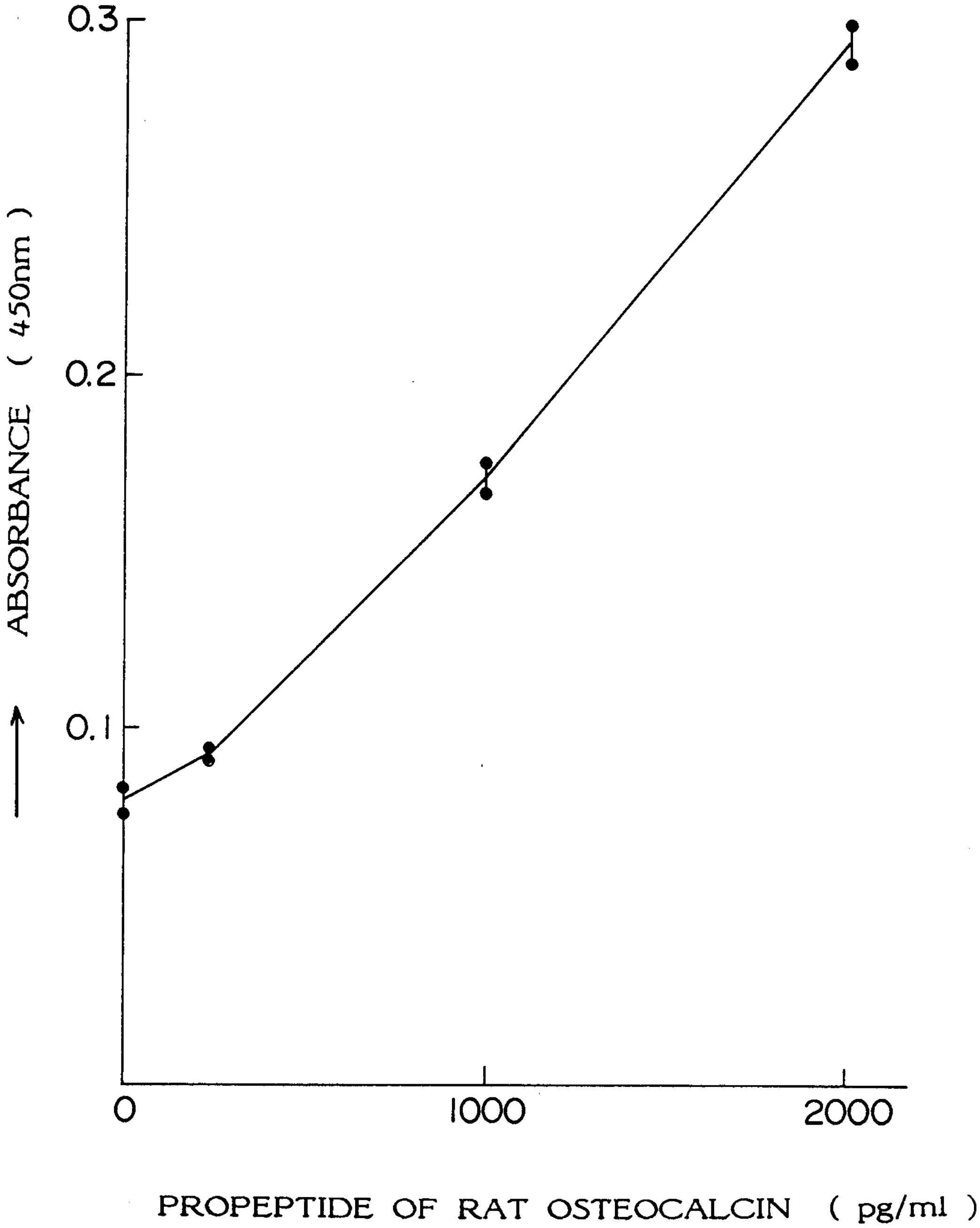
0.3
0.2
0.1
ABSORBANCE ( 450nm )
0
1000
2000
PROPEPTIDE OF RAT OSTEOCALCIN ( pg/ml )

# INTERNATIONAL SEARCH REPORT

International Application No PCT/JP91/01330

**I. CLASSIFICATION OF SUBJECT MATTER** (if several classification symbols apply, indicate all) [6]

According to International Patent Classification (IPC) or to both National Classification and IPC

Int. $Cl^5$ G01N33/53, G01N33/577, G01N33/543, C12P21/08, C12N15/06, C12N5/20

**II. FIELDS SEARCHED**

Minimum Documentation Searched [7]

| Classification System | Classification Symbols |
|---|---|
| IPC | G01N33/53, G01N33/577, G01N33/543, C12P21/08, C12N15/06, C12N5/20 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X,Y | Journal of Japan Bone Metabolism Society, No. 3, Vol. 80, (1990) "Presence of Bone Gla Protein (Osteocalcin) precursor in serum-trial for quantification utilizing engyme antibody technique", Ryuichi Kasai and others, P. 306 | 1-32 |
| Y | The EMBO Journal, Vol. 5, No. 8, (1986), "Isolation of the human gene fore bone gla protein utilizing mouse and rat cDNA clones", p. 1885-1890 | 30-32 |
| Y | JP, A, 62-185169 (K.K. Daiichi Radio Isotope Kenkyusho), August 13, 1987 (13. 08. 87), (Family: none) | 11-28 |
| Y | JP, A, 62-159046 (Zaidan Hojin Kagaku & Kessei Ryoho Kenkyusho), July 15, 1987 (15. 07. 87), (Family: none) | 11-28 |
| Y | JP, A, 61-57856 (Zaidan Hojin Kagaku & | 11-28 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| December 5, 1991 (05. 12. 91) | December 24, 1991 (24. 12. 91) |
| International Searching Authority | Signature of Authorized Officer |
| Japanese Patent Office | |

FURTHER INFORMATION CONTINUED FROM THE SECOND SHEET

| | | |
|---|---|---|
| | Kessei Ryoho Kenkyusho), March 24, 1986 (24. 03. 86) & EP, A1, 173341 | |
| Y | JP, A, 57-124253 (Toyobo Co., Ltd.), August 3, 1982 (03. 08. 82), (Family: none) | 11-28 |
| Y | JP, A, 2-2935 (Tosoh Corp.), January 8, 1990 (08. 01. 90), (Family: none) | 1-28 |
| Y | JP, A, 57-67858 (Takeda Chemical Industries, Ltd.), | 1-28 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

**FURTHER INFORMATION CONTINUED FROM THE SECOND SHEET**

| | | |
|---|---|---|
| | April 24, 1982 (24. 04. 82) & EP, A1, 49898 & US, A, 4496658 | |
| A | JP, A, 2-147954 (Henning Berlin GmbH.), June 6, 1990 (06. 06. 90) & DE, C1, 3833963 | 1-34 |

**V. ☐ OBSERVATIONS WHERE CERTAIN CLAIMS WERE FOUND UNSEARCHABLE [1]**

This international search report has not been established in respect of certain claims under Article 17(2) (a) for the following reasons:

1. ☐ Claim numbers , because they relate to subject matter not required to be searched by this Authority, namely:

2. ☐ Claim numbers , because they relate to parts of the international application that do not comply with the prescribed requirements to such an extent that no meaningful international search can be carried out, specifically:

3. ☐ Claim numbers , because they are dependent claims and are not drafted in accordance with the second and third sentences of PCT Rule 6.4(a).

**VI. ☐ OBSERVATIONS WHERE UNITY OF INVENTION IS LACKING [2]**

This International Searching Authority found multiple inventions in this international application as follows:

1. ☐ As all required additional search fees were timely paid by the applicant, this international search report covers all searchable claims of the international application.
2. ☐ As only some of the required additional search fees were timely paid by the applicant, this international search report covers only those claims of the international application for which fees were paid, specifically claims:
3. ☐ No required additional search fees were timely paid by the applicant. Consequently, this international search report is restricted to the invention first mentioned in the claims; it is covered by claim numbers:
4. ☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.